(19) Europäisches Patentamt / European Patent Office / Office européen des brevets

(11) **EP 1 976 466 B1**

(12) **EUROPÄISCHE PATENTSCHRIFT**

(45) Veröffentlichungstag und Bekanntmachung des Hinweises auf die Patenterteilung:
**20.04.2011 Patentblatt 2011/16**

(21) Anmeldenummer: **07702851.2**

(22) Anmeldetag: **18.01.2007**

(51) Int Cl.:
*A61F 2/84* *(2006.01)*   *A61F 2/06* *(2006.01)*

(86) Internationale Anmeldenummer:
**PCT/EP2007/000403**

(87) Internationale Veröffentlichungsnummer:
**WO 2007/085373 (02.08.2007 Gazette 2007/31)**

(54) **EINFÜHRSYSTEM FÜR STENTS MIT ZUG-DRUCK-KINEMATIK**

INSERTION SYSTEM FOR STENTS, COMPRISING TENSION-COMPRESSION KINEMATICS

SYSTEME D'INSERTION DE PROTHESES INTRAVASCULAIRES A CINEMATIQUE DE TRACTION-COMPRESSION

(84) Benannte Vertragsstaaten:
**DE ES FR GB IT**

(30) Priorität: **25.01.2006 DE 102006004123**

(43) Veröffentlichungstag der Anmeldung:
**08.10.2008 Patentblatt 2008/41**

(73) Patentinhaber: **JOTEC GmbH**
**72379 Hechingen (DE)**

(72) Erfinder:
• **KAUFMANN, Ralf**
**72414 Rangendingen (DE)**
• **DERKVIST, Stefan**
**72379 Hechingen (DE)**
• **HAUSER, Berthold**
**72393 Burladingen (DE)**

(74) Vertreter: **Laufer, Gabriele**
**Witte, Weller & Partner**
**Patentanwälte**
**Postfach 10 54 62**
**70047 Stuttgart (DE)**

(56) Entgegenhaltungen:
EP-A- 1 440 671   US-A- 5 707 376
US-A1- 2003 191 516

**Beschreibung**

Die vorliegende Erfindung betrifft eine Vorrichtung zum Einführen eines selbst expandierenden Stents in ein Körpergefäß mit einem Schlauch, der in einem distalen Abschnitt den Stent radial zusammengedrückt hält, einem in dem Schlauch geführten Schiebeelement mit einem proximalen und einem distalen Ende, ferner einem Griff, der ein Gehäuse aufweist, über welches das Schiebeelement am Griff verschiebbar befestigt ist, ferner mit einem in dem Schiebeelement geführten Stentträger mit Spitze, der über den Griff fest an der Vorrichtung angebracht ist.

Derartige Vorrichtungen zum Einführen von selbstexpandierenden Stents sind im Stand der Technik bekannt. Mit solchen Einführsystemen werden als endovaskuläre Stents bezeichnete Gefäßstents in Blutgefäße implantiert, welche beispielsweise aufgrund von Krankheiten oder Ähnlichem verletzt oder in ihrem Lumen verschlossen sind, wodurch die Gefäße in ihrer Funktion stark beeinträchtigt sind. Im Stand der Technik sind verschiedene implantierbare Stentvorrichtungen bekannt, die Blutgefäße, beispielsweise Arterien, nach ihrer Implantation offen halten. Solche Stents haben in der Regel einen röhrenförmigen Körper, der in das Gefäß eingeführt und an der entsprechenden Stelle fixiert wird, um das Lumen des Gefäßes aufrecht zu erhalten.

So sind im Stand der Technik beispielsweise Stentgrafts bekannt, die ein Drahtgerüst aus einem selbstexpandierenden Material aufweisen, wobei das Drahtgerüst darüber hinaus mit einem Textilschlauch verbunden sein kann.

Zur Implantation wird der Stent radial zusammengedrückt, so dass sich seine Querschnittsfläche deutlich verringert und er einfach in das Gefäß eingeführt werden kann. Aufgrund der Federwirkung des Metallrahmens expandiert der Stent wieder in seine ursprüngliche Form und spannt dabei seine Mantelfläche auf, die sich innen in dem Blutgefäß verklemmt.

Zur Implantation werden die Stents radial zusammengefaltet und dann mit Hilfe von endoluminal vorgeschobenen Kathetern in das Blutgefäß eingeführt und lagerichtig in dem Gefäß positioniert. Die richtige Lage des Stents kann dabei beispielsweise über Röntgenmarker kontrolliert werden. Damit die Stents während der Positionierung im zusammengefalteten Zustand bleiben, sind sie in einer Hülle bzw. in einem hülsenartigen Schlauch angeordnet, der aufgrund seiner Eigenschaft den Stent radial nach innen drückt. Diese so genannte Rückzugshülle wird nach dem Positionieren des stets im Gefäß zurückgezogen, wobei der Stent axial von einem Anschlagrohr gehalten wird, welches auch als "Pusher" bezeichnet wird. Der Pusher liegt dabei in Anlage mit dem Stent und hält diesen in seiner axialen Lage, während die auch den Pusher umgebende Rückzugshülle von dem Stent abgezogen wird, welcher sich dabei expandieren und in dem Blutgefäß verklemmen kann.

Je nach Anwendungsart werden die unterschiedlichsten Stents eingesetzt. Die hier vorliegende Anmeldung beschäftigt sich mit dem Applizieren von so genannten Flechtstents. Diese stellen Metallstents dar, die in so genannter Leinwandbindungstechnik hergestellt werden. Sie bestehen aus einem in Längsrichtung streckbaren, hohlen Körper, dessen Mantel ein Geflecht aus einer Vielzahl von fadenförmigen Elementen ist, die im expandierten Zustand des Flechtstents eine Ebene senkrecht zur Längsrichtung unter einem Flechtwinkel schneiden. Ein Flechtstent erfährt bei seiner Streckung eine große Längenänderung, wobei die Längenänderung umso größer ist, je größer der ursprüngliche Durchmesser und je kleiner der ursprüngliche Flechtwinkel ist.

Zur Implantation wird ein solcher Flechtstent langgestreckt in einem Einführsystem oder Applikator gespeichert, das an einer geeigneten Stelle perkutan in den Körper eingeführt und transluminal bis zu dem Gefäß geführt wird, wo der Stent freigesetzt werden soll.

Bei Stents, die beim Freisetzen keine oder nur eine sehr geringe Längenänderung erfahren, lässt sich die Lage des zu implantierenden Stents beispielsweise über Röntgenmarker problemlos überprüfen.

Bei Flecht(metall)stents taucht jedoch das Problem auf, dass diese sich bei ihrer Freisetzung stark verkürzen. Dabei ist das Verhältnis I/L der Stentlänge I im geladenen Zustand zur freien Stentlänge L von den Durchmessern d im Einführsystem, D im entladenen Zustand sowie vom Flechtwinkel $\alpha$ abhängig:

$$I/L = (D^2 - d^2 \cdot \cos^2\alpha)^{1/2}/(D \sin\alpha).$$

So wird beispielsweise ein Stent mit der Länge L = 40 mm, dem Durchmesser D = 6 mm und einem Flechtwinkel von $\alpha$ = 40°, wenn er auf einen Durchmesser von d = 1,5 mm in einem Einführsystem komprimiert ist, um den Faktor I/L = 1,53 länger. Danach ist er also im Einführsystem 61,2 mm lang. Bei einem Stent mit funktioneller Zone, z.B. mit einem Flechtwinkel von $\alpha$ = 10°, wie er beispielsweise in der Patentanmeldung DE 103 35 649 beschrieben ist, kann die Verlängerung im Einführsystem sogar um den Faktor I/L = 4-6 erfolgen.

Flechtstents sind also extrem streckbar, wobei sie in ihrer langgestreckten Form sozusagen Masse speichern, die beim Zusammenziehen des Stents für einen dichten und steifen, funktionellen Bereich sorgt, wie es ausführlich in der erwähnten DE 103 35 649 beschrieben ist.

Stents, bei denen derartige Verkürzungen bei der Freisetzung berücksichtigt werden müssen, können mit den gegenwärtig existierenden Einführsystemen nicht mehr präzise freigesetzt werden.

Im Stand der Technik sind beispielsweise Einführsysteme für Stents bekannt, die sich beim Freisetzen extrem verkürzen. Bei den im Stand der Technik bekannten Einführsystemen wird das Verkürzungsproblem durch eine Begrenzung des distalen Verfahrwegs der Spitze auf wenige Millimeter gegenüber dem Hüllschlauch gelöst. Bei derartigen Einführsystemen muss das Einführsystem beim Herausdrücken des Stents gleichzeitig vorsichtig zurückgezogen werden. Diese millimetergenaue Positionierung eines solchen sich stark verkürzenden Flechtstents erfordert vom Anwender Übung und sehr viel Erfahrung. Die Präzision und Handhabungseigenschaften dieses Systems sind auf die Behandlung langstreckiger peripherer Gefäßläsionen (größer als 3 cm) mit entsprechend lang geladenen Flechtstents abgestimmt. Für eine millimetergenaue Positionierung in kurzstreckigen Stenosen, wie sie z.B. in der Arteria carotis interna auftreten können, sind solche Systeme zu ungenau.

Die US 2003/0191516 A1 offenbart ein Freisetzungssystem für einen Stent, mit einer äußeren Hülle, die den Stent komprimiert, sowie mit einem Schiebeelement, das an einem Ende des Stents platziert ist, sowie einer Verbindung zwischen dem Schiebeelement und der äußeren Hülle, die die Bewegung der äußeren Hülle in einer ersten Richtung mit einer gleichzeitigen Bewegung des Schiebeelements in eine zweite Richtung koordiniert, wobei letztere der ersten Richtung entgegengesetzt ist. Das in der US 2003/0191516 A1 offenbarte Freisetzungssystem arbeitet dabei mit zwei Zahnstangen, die über zwei Zahnräder miteinander in Verbindung stehen. Die erste Zahnstange ist dabei an ihrem proximalen Ende an den Handgriff gekoppelt.

Das US-Patent US 5,707,376 offenbart eine Einführvorrichtung eines selbstexpandierenden Stents, wobei die Freisetzungsvorrichtung ein äußeres längliches Element mit einer äußeren Passage zur Freisetzung eines selbst expandierenden Stents aufweist, sowie ein inneres längliches Element, das in der äußeren Passage vorgesehen ist, und das in eine zweite Richtung geführt werden kann, um den Stent aus der äußeren Passage freizusetzen. Das äußere und das innere Element sind dabei durch einen Verbindungsmechanismus verbunden, der es ermöglicht, das äußere Element in eine erste Richtung und das innere Element in eine zweite Richtung zu bewegen.

Die EP 1 440 671 A2 offenbart eine Betätigungsvorrichtung für Katheter, wobei diese eine Zahnstange sowie ein mit der Zahnstange zusammenwirkendes Antriebsrad aufweist. Mit dem Antriebsrad ist ferner ein Mechanismus zur Änderung der Geschwindigkeit verbunden, der die Bewegung zwischen einem Reiter und einem beweglichen Element übertragen kann.

Vor diesem Hintergrund liegt der vorliegenden Erfindung die Aufgabe zugrunde, ein Einführsystem bereitzustellen, dass auf konstruktiv einfache Weise Flechtstents mit verbesserter Positionierungsgenauigkeit insbesondere bei kurzstreckigen Stenosen freigesetzt werden können.

Die Erfindung ist in den Ansprüchen definiert.

Bei der eingangs erwähnten Vorrichtung wird diese Aufgabe erfindungsgemäß dadurch gelöst, dass ein im dem Gehäuse des Griffs geführtes bewegliches Element vorgesehen ist, das derart mit dem proximalen Ende des Schiebeelements gekoppelt ist, dass durch eine Bewegung des beweglichen Elements in die proximale Richtung gleichzeitig das Schiebeelement in die distale Richtung, und der Schlauch in die proximale Richtung führbar ist, und wobei das bewegliche Element mit dem Schiebeelement über ein im Griff vorgesehenes Umlenkgetriebe, nämlich über ein Seilrollenumlenkgetriebe, gekoppelt ist.

Die der Erfindung zugrunde liegende Aufgabe wird auf diese Weise vollkommen gelöst.

Die Erfinder der vorliegenden Anmeldung haben nämlich erkannt, dass durch eine Kopplung der Bewegung des Schlauchs bzw. Hüllschlauchs mit dem Schiebeelement bzw. Pusher dafür gesorgt wird, dass der Anwender mit einem einfachen Handgriff eine äußerst präzise Positionierung vornehmen kann. Durch das erfindungsgemäße Einführsystem wird es daher ermöglicht, dass auch weniger erfahrene und geübte Anwender Flechtstents, die sich stark verkürzen, millimetergenau absetzen können.

Mit dem Begriff "distal" wird vorliegend dabei diejenige Richtung/dasjenige Ende der Vorrichtung oder Teile der Vorrichtung bezeichnet, die vom Anwender weg (also in Richtung Spitze des Stentträgers) führt; mit dem Ausdruck "proximal" wird diejenige Richtung/dasjenige Ende der Vorrichtung oder Teile der Vorrichtung bezeichnet, welches zum Anwender hinführt, bzw. hinweist.

Es versteht sich, dass das proximale Ende des Schiebeelements, das sich im Gehäuse befindet, einstückig mit den weiteren Abschnitten des Schiebeelements, die sich zum Teil auch außerhalb des Gehäuses erstrecken, ausgebildet sein kann, oder aus einem oder mehreren getrennten Elementen bestehen kann, die im zusammengebauten Einführsystem mit den weiteren Bestandteilen des Schiebeelements eine Einheit bilden.

Der Stent kann somit durch ein Zurückziehen des Schlauches äußerlich und gleichzeitiges, automatisches, vom Anwender nicht sichtbares Entgegendrücken durch das Schiebeelement bzw. den Pusher kontinuierlich freigesetzt werden. Dies wird durch die bei der erfindungsgemäßen Vorrichtung vorgesehene Kopplung zwischen beweglichem Element und Schiebeelement bewirkt. So kann bspw. durch das Zurückziehen des Schlauches in proximale Richtung das bewegliche Element ebenfalls in eine proximale Richtung geführt werden. Durch die Kopplung mit dem proximalen Ende des Schiebelements wird dabei dann gleichzeitig das Schiebeelement in die distale Richtung bewegt, wodurch der radial zusammengepresste und dadurch stark "verlängerte" Stent in den relativ kurzen Gefäßabsatzbereich freigesetzt werden kann.

Gleichzeitig bleiben der Griff der Vorrichtung und mit ihm die Spitze des Stentträgers während der gesamten Stent-Freisetzung örtlich, also relativ zur Läsion, fixiert. Der Stentträger kann dabei Röntgenmarker aufweisen.

Zu Beginn der Freisetzung des Stents kommt durch die Relativbewegung zwischen Schlauch und Schiebeelement zunächst die Spitze des Stentträgers frei, wobei auch das distale Ende des Stents aus der Außenhülle herausgelangt und sich expandiert, also von der Spitze frei kommt. Durch weiteres Zurückziehen des Hüllschlauchs und durch die mechanisch erzeugte entgegengesetzte Bewegung des Schiebeelements (Pushers) wird der Stent freigesetzt.

"Umlenkgetriebe" soll dabei jedes Bauelement/ Bauelemente bedeuten, mit der eine Bewegung gleichzeitig in eine andere Bewegung umgeleitet wird.

Bei dieser Maßnahme ist von Vorteil, dass bei Einarbeitung eines Umlenkgetriebes in den Griff ein einfach zu handhabendes Bauelement bereitgestellt wird, mit Hilfe dessen Stents, wie oben beschrieben, einfach und genau freigesetzt werden können. Das Umlenkgetriebe bewirkt, dass eine Verschiebung des beweglichen Elements in die proximale Richtung die Bewegung des Schiebeelements in die distale Richtung zur Folge hat.

Ferner ist bevorzugt, wenn das bewegliche Element mit dem im Gehäuse liegenden proximalen Ende des Schiebeelements über ein im Griff vorgesehenes Umlenkgetriebe gekoppelt ist.

Insbesondere ist bevorzugt, wenn das proximale Ende des Schiebeelements einen Flansch aufweist, über welchen das bewegliche Element mit dem Schiebeelement gekoppelt ist.

Diese Maßnahme hat den Vorteil, dass durch den Flansch eine geeignete Fläche vorgesehen wird, an welcher beispielsweise Elemente des Umlenkgetriebes befestigt sein können. An dem Flansch kann - je nach Ausführung des Umlenkgetriebes - bspw. der Zugfaden mit einem seiner Enden angebracht sein, und/oder über ein am Flansch vorgesehenes Umlenkelement geführt werden.

Dabei ist insbesondere bevorzugt, wenn der Flansch derart dimensioniert und im Griff vorgesehen ist, dass Elemente des Flansches außerhalb des Gehäuses greifbar sind. Flansch und Schiebeelement bilden vorzugsweise eine Baueinheit.

Diese Maßnahme hat den Vorteil, dass über die außerhalb des Griffes hinausragenden Elemente des Flansches das Schiebeelement unabhängig von der Bewegung des beweglichen Elements geführt werden kann. Dazu muss lediglich an die äußeren Elemente des Flansches gegriffen werden, die über das Gehäuse des Griffes hinausragen. Es versteht sich, dass an dem Flansch und zusätzlich zu den aus dem Gehäuse herausragenden Enden noch zusätzliche Griffelemente vorgesehen sein können. Dabei können beispielsweise die Elemente des Flansches derart ausgebildet sein, dass ein möglichst sicheres und rutschfestes Greifen des Flansches und damit indirekt auch des Schiebeelements ermöglicht wird. Hierzu können die Elemente des Flansches beispielsweise eine "geriffelte" oder sonstige raue Oberfläche aufweisen, die ein Verrutschen des Griffs auf dem Flansch sowie ein leichteres manuelles Führen des Schiebeelements ermöglichen.

Bei einer Ausführungsform der erfindungsgemäßen Vorrichtung ist bevorzugt, wenn im Gehäuse ferner ein Zuggriff vorgesehen ist, der mit dem Schlauch gekoppelt ist.

Diese Ausführungsform hat den Vorteil, dass das bewegliche Element zunächst durch die Bewegung des Zuggriffs, der mit dem Schlauch gekoppelt ist, in die proximale Richtung verschoben werden kann, bzw. durch die konstruktive Anordnung des beweglichen Elements im Griff, welches mit dem Zuggriff in Reihe angeordnet, bzw. dem Zuggriff - in proximaler Richtung gesehen - nachgeschaltet oder nachgeordnet ist, mitgenommen wird. Das dadurch in Bewegung versetzte bewegliche Element bewirkt wiederum durch die Kopplung - bspw. über ein Umlenkgetriebe - die Bewegung des Schiebeelements in die distale Richtung.

Die Freisetzung des Stents wird dadurch einerseits durch Zurückziehen des den Stent zusammendrückenden Schlauchs bewirkt und andererseits durch aktives Entgegenwirken und -schieben durch das Schiebeelement.

Dabei ist das Schiebeelement derart im Gehäuse des Griffs angeordnet, dass das bewegliche Element und der Zuggriff entlang des Schiebeelements geführt werden, bzw. das Schiebeelement in distale Richtung zumindest teilweise durch das bewegliche Element und den Zuggriff hindurch verschiebbar angeordnet ist.

In einer Ausführungsform des erfindungsgemäßen Einführsystems ist bevorzugt, wenn das Umlenkgetriebe einen Zugfaden aufweist, der mit dem einen seiner Enden am distalen Teil des Gehäuses befestigt ist, der ferner über ein erstes am beweglichen Element vorgesehenes Umlenkelement und über ein zweites im distalen Ende des Gehäuses vorgesehenes Umlenkelement zum Schiebeelement geführt und mit einem zweiten Ende am Schiebeelement befestigt ist.

Diese Ausführungsform hat den Vorteil, dass mit dem Umlenkgetriebe ein Übersetzungsverhältnis X/Y von 0,5 erreicht werden kann, wobei X der Weg des beweglichen Elements bezogen auf den Weg Y des Schiebeelements ist.

In einer anderen Ausführungsform der erfindungsgemäßen Vorrichtung ist bevorzugt, wenn das Umlenkgetriebe einen Zugfaden aufweist, der mit dem einen seiner Enden am beweglichen Element befestigt ist, der ferner über ein am distalen Ende des Gehäuses vorgesehenes Umlenkelement zum Schiebeelement geführt und mit seinem zweiten Ende am Schiebeelement befestigt ist.

Bei dieser Ausführungsform kann vorteilhafterweise ein Übersetzungsverhältnis von X/Y von 1 erreicht werden.

In einer weiteren Ausführungsform ist bevorzugt, wenn das Umlenkgetriebe einen Zugfaden aufweist, der mit dem einen seiner Enden am beweglichen Element befestigt ist, der ferner über ein erstes am distalen Ende des Gehäuses vorgesehenes Umlenkelement zum Schiebeelement geführt ist, der über ein zweites, am Schiebeelement vorgesehenes Umlenkelement wieder zum distalen Ende des Gehäuses geführt und mit seinem zweiten Ende am distalen Ende des Gehäuses befestigt ist.

Diese Ausführungsform hat den Vorteil, dass mit dieser ein Übersetzungsverhältnis von X/Y von 2 erreicht werden kann.

Wie oben dargestellt, können durch die unterschiedlichen Ausführungen des Umlenkgetriebes verschiedene Übersetzungsverhältnisse generiert werden, die je nach einzuführendem Stent, bzw. Gefäßlage oder Erfahrenheit des Anwenders, für den jeweiligen spezifischen Einsatz ausgewählt werden können.

Dabei wird die Wahl der Ausführungsform insbesondere von dem Wert des Faktors I/L des Stents oder einzelnen Stentzonen abhängig gemacht werden (Stentlänge I im geladenen Zustand im Verhältnis zur freien Stentlänge L).

Danach kann aber auch beispielsweise der anfängliche Rückzugsweg Z des Schlauches variiert und dem jeweils vorliegenden Stentdurchmesser und Stentwinkel angepasst werden. Dadurch können beispielsweise auch irrationale Zahlen als Gesamtübersetzungsverhältnisse $(Z+X)/Y \geq 0,5$ zwischen Rückzugsweg des Schlauches und Vorwärtsbewegung des Schiebeelements ermöglicht werden. Die gleiche Wirkung hat ein um die Länge Z gelockerter Zugfaden und die Vereinigung der Bauelemente.

Es versteht sich, dass als "Zugfaden" jedes fadenförmige Element geeignet ist, gleich welcher Beschaffenheit oder gleich welchem Material, das die für ein Umlenkgetriebe erforderlichen Merkmale wie oben beschrieben umzusetzen vermag. Der Zugfaden muss daher ein hohes Elastizitätsmodul bei guter Biegsamkeit und geringer Reibung aufwiesen. Bspw. kann ein geflochtenes Textilseil eingesetzt werden. Gleiches gilt für die Umlenkelemente. Diese können bspw. als kleine Rollen ausgebildet sein, über die der Zugfaden geführt wird. Auch für die Befestigung der Enden des Zugfadens stehen verschiedene Möglichkeiten zur Auswahl, wie bspw. haken- oder ösenförmige Elemente, ohne jedoch hierauf beschränkt zu sein.

In einer bevorzugten Ausführungsform weist die Vorrichtung ein weiteres Getriebe auf, das als Gegengetriebe zu dem Umlenkgetriebe arbeitet.

Diese Ausführungsform ist insbesondere dann vorteilhaft, wenn der Anwender den Stent durch Zusammenschieben des beweglichen Elements und des Flansches freisetzen will: Durch das Gegengetriebe wird sichergestellt, dass sich der Zugfaden nicht lockern kann, und dass das vorgesehene Getriebeübersetzungsverhältnis zwischen Züggriff und beweglichem Element nicht außer Kraft gesetzt wird. Umlenkgetriebe und Gegengetriebe haben das gleiche Getriebeübersetzungsverhältnis. Wenn der Zugfaden des Umlenkgetriebes auf Druck beansprucht wird und lose kommt, wird das Gegengetriebe auf Zug beansprucht, und umgekehrt. Dadurch bleibt das Getriebeübersetzungsverhältnis zwischen Zuggriff und beweglichem Element stets erhalten, unabhängig davon, welches Element oder welche Elementkombination vom Anwender betätigt wird.

In weiteren Ausführungsformen des erfindungsgemäßen Einführsystems bzw. der erfindungsgemäßen Vorrichtung ist bevorzugt, wenn der Griff ferner ein Greifelement aufweist, das fest am Gehäuse angebracht ist.

Bei dieser Maßnahme ist von Vorteil, dass durch das Greifelement ein sicheres Greifen des Griffs bzw. des Gehäuses und damit auch der gesamten Vorrichtung ermöglicht wird. Demnach kann beispielsweise das Greifelement als eine Art Fingerloch bzw. Daumenloch ausgebildet sein, in welches der Anwender lediglich einen entsprechenden Finger einführen muss. Die Hand des Anwenders kann dabei dann gleichzeitig den Griff umgreifen. Das Einführsystem kann einfach mit einer Hand betätigt werden: hierzu greift der Anwender mit dem Daumen in das Greifelement, wodurch er den gesamten Griff des Einführsystems zu greifen bekommt. Über das Greifelement ist der Griff in der Hand des Anwenders fixiert; soll der Stent nach einer Einführung in das entsprechende Gefäß freigesetzt werden, kann der Anwender bspw. mit Zeige- und/oder Mittelfinger den Züggriff greifen und diesen in eine proximale Richtung führen, wodurch der Stent - wie oben beschrieben - kontinuierlich freigesetzt wird.

In einer Ausführungsform der erfindungsgemäßen Vorrichtung ist vorgesehen, dass der Anfangsabstand zwischen Zuggriff und beweglichem Element $\geq 0$ mm ("größer oder gleich null") ist.

Mit "Anfangsabstand" ist dabei vorliegend der Abstand zwischen Zuggriff und beweglichem Element gemeint, der nach Einführen der Vorrichtung in ein Gefäß und somit zu Beginn des Freisetzungsmechanismus vorliegt.

Bei einer weiteren Ausführungsform der erfindungsgemäßen Vorrichtung ist vorgesehen, dass das bewegliche Element als Zugelement, insbesondere als flanschförmiges Zugelement, ausgebildet ist, welches auch mit dem Schlauch gekoppelt ist.

Mit dieser Ausführungsform wird erreicht, dass damit eine technisch einfache Variante der erfindungsgemäßen Vorrichtung bereitgestellt wird, da durch das Zurückziehen des beweglichen Elements bzw. Zugelements gleichzeitig bzw. simultan sowohl die Hülle vom Stent zurückgezogen wird, also in proximale Richtung bewegt wird, als auch das Schiebeelement in distale Richtung bewegt wird, wodurch der Stent durch eine weitere Krafteinwirkung freigesetzt werden kann.

Durch die Ausbildung des beweglichen Elementes als Flansch wird erreicht, dass - wie bei der Ausbildung des proximalen Endes des Schiebeteils als Flansch - durch den Flansch eine geeignete Fläche bereitgestellt wird, an welcher beispielsweise Elemente des Umlenkgetriebes befestigt sein können. An dem Flansch kann - je nach Ausführung des Umlenkgetriebes - bspw. der Zugfaden mit einem seiner Enden angebracht sein, und/oder über ein am Flansch vorgesehenes Umlenkelement geführt werden.

Dabei ist bei einer bevorzugten Weiterbildung der Ausführungsform bevorzugt, wenn das Umlenkgetriebe einen Zugfaden aufweist, der mit dem einen seiner Enden am distalen Teil des Gehäuses befestigt ist, der ferner über ein erstes, am Zugelement vorgesehenes Umlenkelement und über ein zweites, im distalen Ende des Gehäuses vorgesehenes Umlenkelement, zu einem am proximalen Ende des Gehäuses vorgesehenen Umlenkelement geführt ist, und anschließend über ein am proximalen Ende des Schiebelements vorgesehenes Mittel zum Fixieren des Zugfadens am proximalen Ende des Schiebeelements wieder um das Umlenkelement geführt ist und mit seinem zweiten Ende am proximalen Ende des Gehäuses befestigt ist.

Diese Ausführungsform hat den Vorteil, dass mit dem Umlenkgetriebe ein Übersetzungsverhältnis X/Y von 0,5 erreicht werden kann, wobei X der Weg des beweglichen Elements bzw. Zugelements bezogen auf den Weg Y des Schiebeelements ist.

Bei einer anderen Weiterbildung der Ausführungsform ist bevorzugt, wenn das Umlenkgetriebe einen umlaufenden Zugfaden aufweist, der über ein am distalen Ende des Gehäuses vorgesehenes Umlenkelement, über ein am Zugelement vorgesehenes Mittel zum Fixieren des Zugfadens am Zugelement, über ein am proximalen Ende des Gehäuses vorgesehenes Umlenkelement und über ein am proximalen Ende des Schiebeelements vorgesehenes zweites Mittel zum Fixieren des Zugfadens am proximalen Ende des Schiebeelements geführt ist.

Bei dieser Weiterbildung der Ausführungsform kann vorteilhafterweise ein Übersetzungsverhältnis von X/Y von 1 erreicht werden.

Bei einer noch anderen Weiterbildung der Ausführungsform ist bevorzugt, wenn das Umlenkgetriebe einen Zugfaden aufweist, der mit dem einen seiner Enden am distalen Ende des Gehäuses befestigt ist und über ein am proximalen Ende des Schiebeelements vorgesehenes Umlenkelement, über ein am distalen Ende des Gehäuses vorgesehenes Umlenkelement, über ein am Zugelement vorgesehenes Mittel zum Fixieren des Zugfadens am Zugelement, und über ein am proximalen Ende des Gehäuses vorgesehenes Umlenkelement geführt ist, und von dort wieder zum Umlenkelement, und der mit seinem zweiten Ende am proximalen Ende des Gehäuses befestigt ist.

Mit dieser Weiterbildung der Ausführungsform kann ein Übersetzungsverhältnis von X/Y von 2 erreicht werden.

Unter "Umlenkelement" wird jedes Bauelement verstanden, das dazu geeignet ist, die durch den Zugfaden in eine Richtung ausgeübte Bewegung oder Kraft umzulenken. So sind bspw. Umlenkelemente in Form von festen oder beweglichen Rollen bevorzugt.

Unter "Mittel zum Fixieren des Zugfadens" am proximalen Ende des Schiebeelements oder am Zugelement wird jedes Maßnahme verstanden, mit der der Zugfaden an dem Schiebeelement oder Zugelement derart fixiert wird, dass, wenn das Zugelement/Schiebeelement bewegt wird, der Zugfaden durch dessen Fixierung über die vorgesehenen Mittel mitgezogen wird. Die "freien" Abschnitte des Zugfadens, also diejenigen, die nicht über die Mittel fixiert sind, können dann über die Umlenkelemente laufen. Solche "Mittel zum Fixieren" können bspw. Klemmelemente sein. Der Zugfaden kann aber auch durch andere technische Verfahren am Zugelement/Schiebeelement fixiert werden, wie bspw. Kleben, Verschweißen oder ähnliches.

Bei den oben beschriebenen Weiterbildungen ist bevorzugt, wenn das Zugelement und/oder das Schiebelement Elemente aufweisen, die außerhalb des Gehäuses greifbar sind. Diese Maßnahme hat den Vorteil, dass über die außerhalb des Gehäuses des Griffes hinausragenden Elemente das Schiebeelement und das beweglichen Element/Zugelement betätigt werden können. Auch hier versteht sich, dass zusätzlich zu den aus dem Gehäuse herausragenden Elementen noch zusätzliche Griffelemente vorgesehen sein können. Durch die Ausbildung von Griffelementen in Form von bspw. eines "Abzuges", kann das bewegliche Element und/oder das Schiebeelement einfach durch Betätigung des Abzuges mit einem Finger des Anwenders in Bewegung gesetzt werden.

In einer weiteren Ausführungsform ist bevorzugt, wenn die Vorrichtung ferner ein Stoppelement aufweist, welches verschiebbar zwischen dem beweglichen Element und dem Schiebeelement vorgesehen ist.

Diese Maßnahme hat den Vorteil, dass dadurch verhindert wird, dass das bewegliche Element zunächst direkt an das Schiebeelement anstößt. Dadurch wird wiederum sichergestellt, dass ein ausreichender Teil des proximalen Stentendes im Schlauch liegen und am Schiebeelement fixiert bleibt.

Das Stoppelement ist dabei in Reihe mit dem Zuggriff und dem beweglichen Element angeordnet. Durch die Bewegung des Zuggriffs wird somit zunächst das bewegliche Element in Bewegung versetzt, wobei das bewegliche Element auch bspw. direkt durch Bewegen des Zuggriffs in proximale Richtung verschoben werden kann. Das bewegliche Element stößt dann - entweder direkt oder im Verlauf seiner Bewegung - an das Stoppelement, das durch die Bewegung des beweglichen Elements in proximale Richtung mitgeführt wird. Durch die Kopplung des beweglichen Elements mit dem Schiebeelement wird dieses - wie oben beschrieben - in die distale Richtung geführt, wodurch es dem beweglichen Element und dem Stoppelement entgegenkommend bewegt wird. Je nach Ausführungsform des Umlenkgetriebes stoßen dann nach einer bestimmten Weglänge das durch die Bewegung des beweglichen Elements mitgeführte Stoppelement und das Schiebeelement aufeinander.

Das Stoppelement kann dabei auch direkt am beweglichen Element entfernbar angebracht sein.

In einer anderen Ausführungsform kann das Stoppelement auch als zwar nicht verschiebbares Element, jedoch als entfernbares Element im bzw. am Griff angebracht sein.

Aufgrund des Stoppelements besteht daher vorteilhafterweise die Möglichkeit, den Stent wieder vollständig in das Einführsystem zurückzuziehen, sollte dies in einer entsprechenden Situation gewünscht werden. Das Zurückziehen des Stents in den Schlauch ist daher sogar in der Position möglich, in welcher das bewegliche Element über das Stoppelement an das Schiebeelement anstößt. Hierfür kann dann das Schiebeelement in proximale Richtung gezogen werden, beispielsweise über seinen Flansch, der außerhalb des Gehäuses greifbar ist. Durch die Bewegung des Schiebeelements in die proximale Richtung wird wiederum das bewegliche Element (zurück) in die distale Richtung bewegt, wodurch auch der Zuggriff und der mit dem Zuggriff gekoppelte Schlauch in die distale Richtung bewegt werden. Dadurch kann der Stent wieder in das Einführsystem geführt werden.

Insgesamt ist dabei bevorzugt, wenn das Stoppelement zwischen beweglichem Element und Schiebeelement entfernbar angebracht ist.

Diese Maßnahme hat den Vorteil, dass der Stent dann endgültig freigesetzt werden kann, wozu das Stoppelement zwischen dem beweglichen Element und dem Schiebeelement einfach entfernt - bspw. herausgezogen - werden kann. Auf diese Weise kann dann in einem nächsten Schritt das bewegliche Element vollständig nach proximal bewegt und in direkten Kontakt mit dem Schiebeelement gebracht werden, wodurch schließlich das proximale Ende des Stents vollständig freigesetzt wird.

Bei der erfindungsgemäßen Vorrichtung ist dabei das Schiebeelement vorzugsweise durch das Stoppelement, das bewegliche Element und den Zuggriff hindurchgeführt. Alle Elemente, also das Schiebeelement, das bewegliche Element, das Stoppelement und der Zuggriff, können dabei gegeneinander verschoben werden, bzw. sind verschiebbar gegeneinander angebracht, wobei - wie beschrieben - das Schiebeelement durch die anderen Elemente verschiebbar hindurchgeführt ist.

Weitere Vorteile und Merkmale ergeben sich aus der nachfolgenden Beschreibung und den beigefügten Zeichnungen.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Ausführungsbeispiele der Erfindung sind in der Zeichnung dargestellt und werden in der nachfolgenden Beschreibung näher erläutert. Es zeigen:

Fig. 1      die Gesamtansicht eines Einführsystems mit geladenem Stent, wobei die Darstellung nicht maßstabsgetreu sein soll;

Fig. 2a      einen vergrößerten, schematischen und nicht maßstabsgetreuen Längsschnitt durch den proximalen Abschnitt des Einführsystems aus Fig. 1 in einem ersten Ausführungsbeispiel;

Fig. 2b      den gleichen Längsschnitt wie in Fig. 2a, wobei in Fig. 2b ein weiteres Getriebe vorgesehen ist;

Fig. 3a      in einer Darstellung wie Fig. 2 ein zweites Ausführungsbeispiel des neuen Einführsystems;

Fig. 3b      den gleichen Längsschnitt des Ausführungsbeispiels wie in Fig. 3a, wobei in Fig. 3b ein weiteres Getriebe vorgesehen ist;

Fig. 4a      in einer Darstellung wie Fig. 2 und 3 ein drittes Ausführungsbeispiel des neuen Einführsystems;

Fig. 4b      den gleichen Längsschnitt des Ausführungsbeispiels wie in Fig. 4a, wobei in Fig. 4b ein weiteres Getriebe vorgesehen ist;

Fig. 5a      eine schematische, perspektivische und nicht maßstabsgetreue Darstel- lung einer anderen Ausführungsform des proximalen (Griff-)Abschnitts des erfindungsgemäßen Einführsystems, im unbetätigten Zustand und somit vor Freisetzung des Stents (letzterer nicht gezeigt), wobei zur bes- seren Darstellung des Inneren des Gehäuses das Gehäuse nur teilweise dargestellt ist;

Fig. 5b      eine schematische Darstellung des Verlaufes des Zugfadens;

Fig. 5c      die Ausführungsform aus Fig. 5a, wobei hier das bewegliche Element und das Schiebeelement durch eine Betätigung der Vorrichtung zu- sammengeführt sind, wodurch der Stent freigesetzt wird (letzterer nicht gezeigt).

Fig. 6a      eine schematische, perspektivische und nicht maßstabsgetreue Darstel- lung einer noch anderen Ausführungsform des proximalen (Griff-) Ab- schnitts des erfindungsgemäßen Einführsystems, im unbetätigten Zu- stand und somit vor Freisetzung des Stents (letzterer nicht gezeigt), wo- bei zur besseren Darstellung des Inneren des Gehäuses das Gehäuse nur teilweise dargestellt ist;

Fig. 6b      die Ausführungsform aus Fig. 6a, wobei hier das bewegliche Element und das Schiebeelement durch eine Betätigung der Vorrichtung zu- sammengeführt sind, wodurch der Stent freigesetzt wird (letzterer nicht gezeigt).

Fig. 7a    eine schematische, perspektivische und nicht maßstabsgetreue Darstel- lung noch einer weiteren Ausführungsform des proximalen (Griff-) Ab- schnitts des erfindungsgemäßen Einführsystems, im unbetätigten Zu- stand und somit vor Freisetzung des Stents (letzterer nicht gezeigt), wo- bei zur besseren Darstellung des Inneren des Gehäuses das Gehäuse nur teilweise dargestellt ist;

Fig. 7b    eine schematische Darstellung des Verlaufes des Zugfadens;

Fig. 7c    die Ausführungsform aus Fig. 7a, wobei hier das bewegliche Element und das Schiebeelement durch eine Betätigung der Vorrichtung zu- sammengeführt sind, wodurch der Stent freigesetzt wird (letzterer nicht gezeigt).

Fig. 8A    eine perspektivische Draufsicht auf den Griff des Einführsystems (a) sowie die Seitenansicht des distalen Abschnitts des Einführsystems (b) in einer Position, in welcher der Stent sich in einer vom Schlauch zu- sammengedrückten Form befindet;

Fig. 8B    eine Darstellung wie in Fig. 8A, wobei der Stent am distalen Ende um eine kurze Strecke freigesetzt ist;

Fig. 8C    eine Darstellung wie in Fig. 8A, wobei hier der nächste Schritt der Stentfreisetzung gezeigt ist;

Fig. 8D    eine Darstellung wie in Fig. 8A, wobei das Stoppelement das direkte Anstoßen des be-weglichen Elements mit dem Schiebe-elementanschlag verhindert; und

Fig. 8E    eine Darstellung wie in Fig. 8A, wobei hier jedoch das Stoppelement entfernt wurde, wodurch das bewegliche Element vollständig nach proximal bis zum Anstoßen auf das Schiebeelement zurückgezogen und der Stent dadurch freigesetzt werden kann.

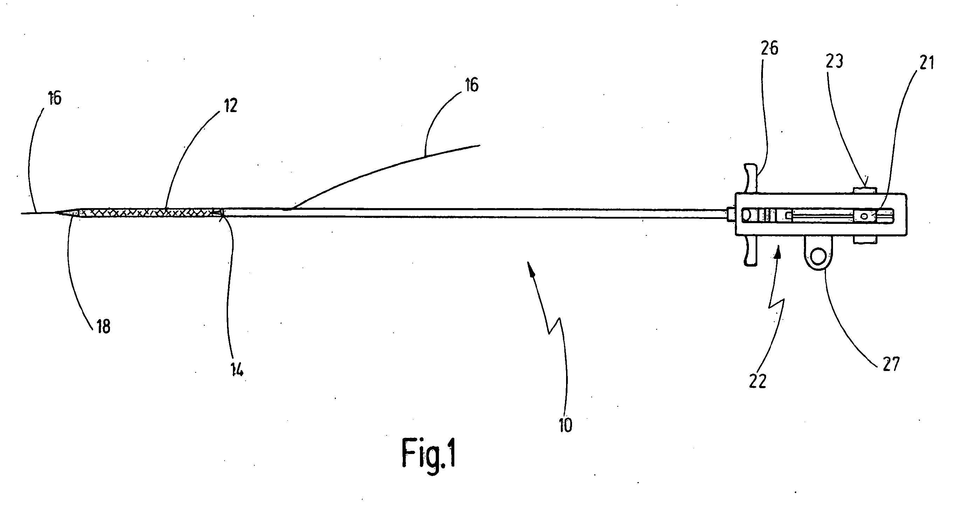

In Fig. 1 ist mit 10 insgesamt - schematisch - ein Einführsystem bezeichnet, mit dem ein mit 12 angedeuteter Stent, bspw. ein Flechtstent, in ein Blutgefäß eingeführt werden kann.

Der Stent 12 kann dabei ein selbstexpandierender Metallstent sein, der in Leinwandbindungstechnik hergestellt ist, wie dies in der eingangs erwähnten DE 103 35 649 beschrieben ist.

Das Einführsystem 10 weist ferner einen Schlauch 14 auf, der den Stent 12 in dem distalen Teil des Schlauches 14 radial zusammengedrückt hält. In diesem Zustand wird die Vorrichtung 10 in ein Gefäß eingebracht und an die gewünschte Position zur Stützung des Gefäßes durch den Stent platziert.

Das Einführsystem aus Fig. 1 weist ferner einen Griff 22 auf, an welchem ein Zuggriff 26 sowie ein Daumenloch 27 vorgesehen sind. Ferner sind Enden des Flansches 21 angedeutet, die eine geriffelte Oberfläche 23 aufweisen. Ferner ist in Fig. 1 ein Führungsdraht 16 angedeutet. Mit diesem Führungsdraht 16 wird gemäß der an sich bekannten Seldinger-Methode das Einführsystem 10 in ein Blutgefäß eines Patienten eingeführt, um dort den Stent 12 freizusetzen.

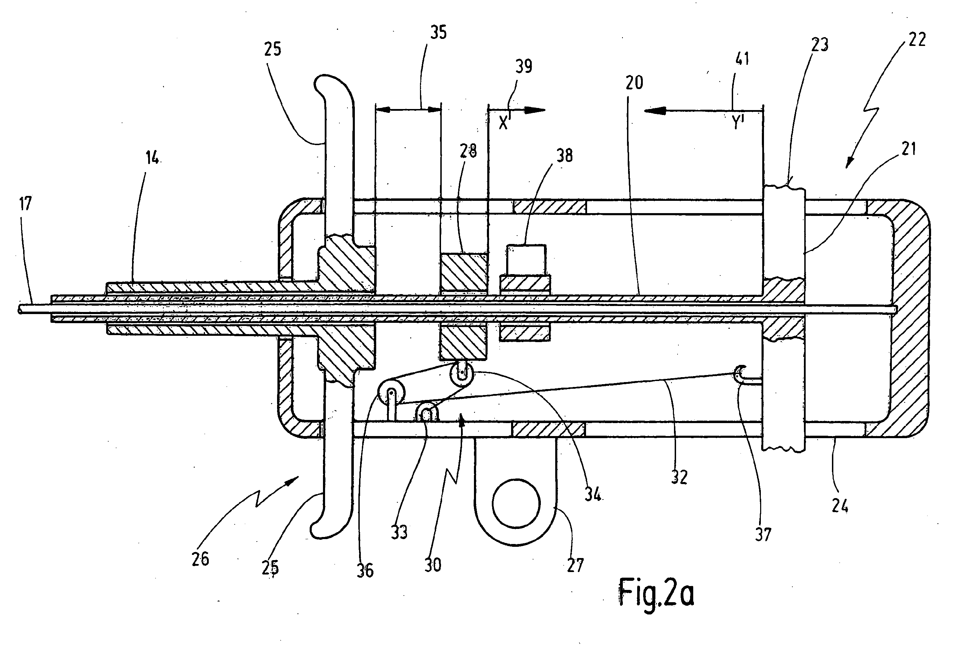

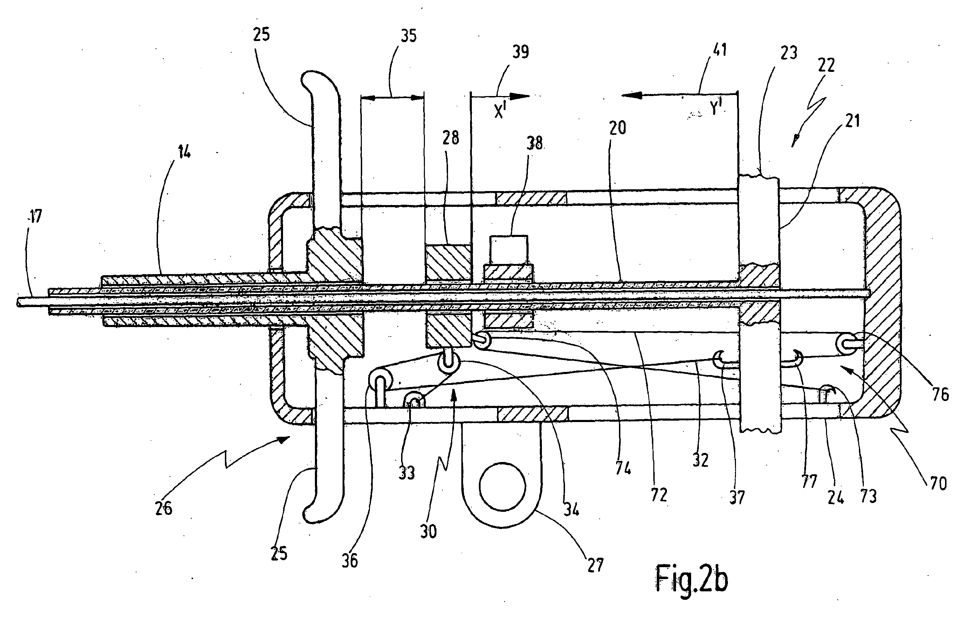

In den Fig. 2a und 2b ist das proximale Ende des Einführsystems in einer ersten Ausführungsform in einem schematischen Längsschnitt vergrößert gezeigt.

Dabei sind gleiche Elemente wie in Fig. 1 mit gleichen Bezugszeichen gekennzeichnet.

In den Fig. 2a und 2b ist mit 22 insgesamt der Griff des Einführsystems bezeichnet, der ein Gehäuse 24 aufweist. Ferner ist mit 26 ein Zuggriff bezeichnet, der in dem Gehäuse 24 führbar angeordnet ist und der direkt mit dem Schlauch 14 gekoppelt ist. Ferner ist in dem Gehäuse ein verschiebbares Schiebeelement 20 vorgesehen, welches bei Stent-Einführungssystemen allgemein auch als Pusher bezeichnet wird. Im Schiebeelement 20 ist ein Stentträger 17 angeordnet, der über den Griff 22 fest an der Vorrichtung 10 verankert ist. Der Stentträger 17 weist an seinem distalen Ende eine atraumatische Spitze 18 auf. In dem Stentträger 17 ist der Führungsdraht 16 geführt.

In dem Gehäuse 24 des Griffs 22 ist weiterhin ein in dem Griff 22 führbares bewegliches Element 28 vorgesehen, welches entlang des sich durch das Gehäuse 24 des Griffs 22 erstreckenden Schiebeelementes 20 verschiebbar angeordnet ist.

Das bewegliche Element 28 ist in Fig. 2a und Fig. 2b über ein SeilrollenUmlenkgetriebe 30 mit dem Schiebeelement 20 gekoppelt. Das Gehäuse 24 des Griffs 22 fungiert insgesamt als Festlagerbasis für die nachstehenden noch genauer zu beschreibenden Um-lenk- und Haltefunktionen des Seilrottenumtenkgetrie-bes. Das Schiebeelement 20 weist an seinem proximalen Ende einen Flansch 21 auf, der wiederum teilweise aus dem Gehäuse 24 des Griffs 22 herausragt. Die aus dem Gehäuse 24 herausragenden Enden des Flanschs 21 sind mit 23 bezeichnet.

Wie Fig. 2a und Fig. 2b zu entnehmen ist, bewirkt die Kopplung zwischen dem beweglichen Element 28 mit dem Schiebeelement 20 durch das Umlenkgetriebe 30 eine Vorschubbewegung des Schiebeelements 20 nach distal.

In Fig. 2a und Fig. 2b weist das Umlenkgetriebe 30 einen Zugfaden 32 auf, der mit dem einen seiner Enden am distalen Teil des Gehäuses 24 über ein Element nach Art einer Öse 33 befestigt ist. Der Zugfaden 32 wird über ein erstes, am beweglichen Element 28 vorgesehenes Umlenkelement 34 und über ein zweites, im distalen Ende des Gehäuses 24 vorgesehenes Umlenkelement 36 zum Schiebeelement 20 geführt. Das Umlenkelement 34 und das Umlenkelement 36 sind dabei beispielhaft als Rollen ausgebildet. Der Zugfaden 32 ist dann mit seinem zweiten Ende über ein Hakenelement 37 am Schiebeelement 20 befestigt. Das Hakenelement 37 befindet sich dabei am Flansch 21 des Schiebeelements 20, und zwar in einem Teil des Flansches 21, der sich innerhalb des Gehäuses 24 befindet.

Mit der in Fig. 2a und 2b teilweise gezeigten Ausführungsform des erfindungsgemäßen Einführsystems, bzw. mit dessen Umlenkgetriebe 30, wird ein Übersetzungsverhältnis X'/Y' von 0,5 erreicht, wobei X' der Weg des beweglichen Elements 28 bezogen auf den Weg Y' des proximalen Endes des Schiebeelements 20, also des Flansches 21, ist. Dieses Verhältnis ist durch die Pfeile 39 und 41 angedeutet. Der Pfeil 35 bezeichnet den Weg Z, der den anfänglichen Rückzugsweg des Schlauchs 14 anzeigt.

In Fig. 2b ist ferner ein weiteres Getriebe 70 gezeigt, das als Gegengetriebe des Seilrollenumlenkgetriebes 30 arbeitet. Dieses Getriebe 70 kann vorgesehen sein, damit der Anwender auch die Möglichkeit hat, den Stent durch Verschieben des beweglichen Elements 28 nach distal wieder zurückladen zu können. Auch wird durch das weitere Getriebe 70 dem Anwender die Möglichkeit geboten, den Stent durch Zusammenschieben des beweglichen Elements 28 mit dem Flansch 21 freizusetzen.

Auch das Getriebe 70 weist einen Zugfaden 72 auf, der mit dem einen seiner Enden am proximalen Ende des Gehäuses 24 über ein Ösenelement 73 befestigt ist. Der Zugfaden 72 wird über ein am beweglichen Element 28 vorgesehenes Umlenkelement 74 und über ein weiteres, am proximalen Ende des Gehäuses 24 vorgesehenes Umlenkelement 76 zum Schiebeelement 20 geführt. Die Umlenkelemente 74, 76 des Getriebes 70 sind dabei beispielhaft als Rollen ausgebildet. Der Zugfaden 72 ist mit seinem zweiten Ende über ein Hakenelement 77 am Schiebeelement 20 befestigt. Das Hakenelement befindet sich dabei am Flansch 21 des Schiebeelements 20, und zwar im Teil des Flansches 21, der sich innerhalb des Gehäuses 24 befindet.

Dem in Fig. 2b gezeigten Aufbau der erfindungsgemäßen Ausführungsform ist zu entnehmen, dass das Gegengetriebe 70 auf Zug beansprucht wird, wenn der Zugfaden 32 des Seilrollenumlenkgetriebes 30 auf Druck beansprucht wird und lose kommt. Auf diese Weise bleibt das Getriebeübersetzungsverhältnis zwischen Zuggriff 26 und beweglichen Element 28 stets erhalten, unabhängig davon, welches Element oder welche Elementkombination betätigt wird.

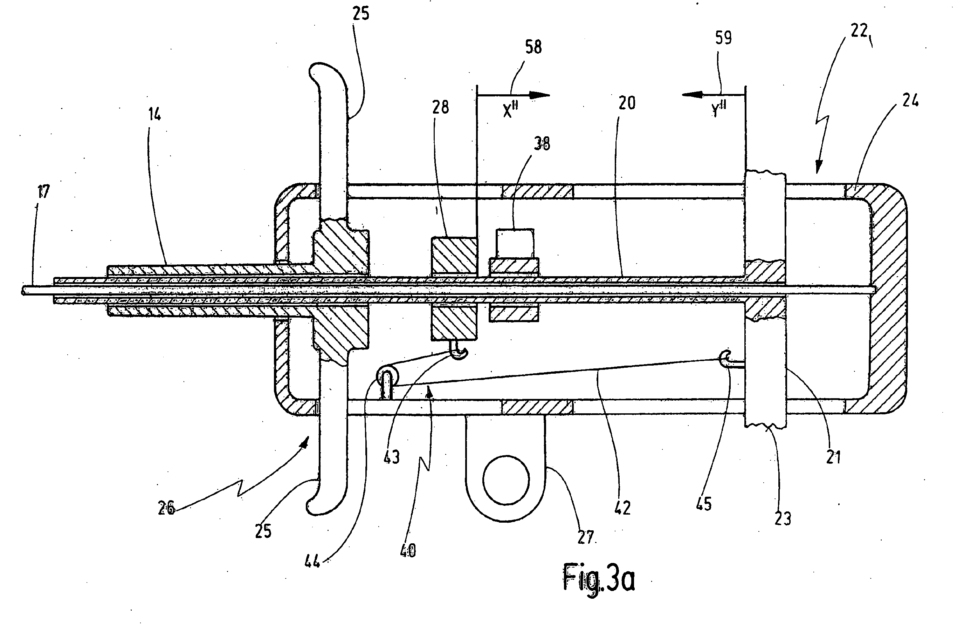

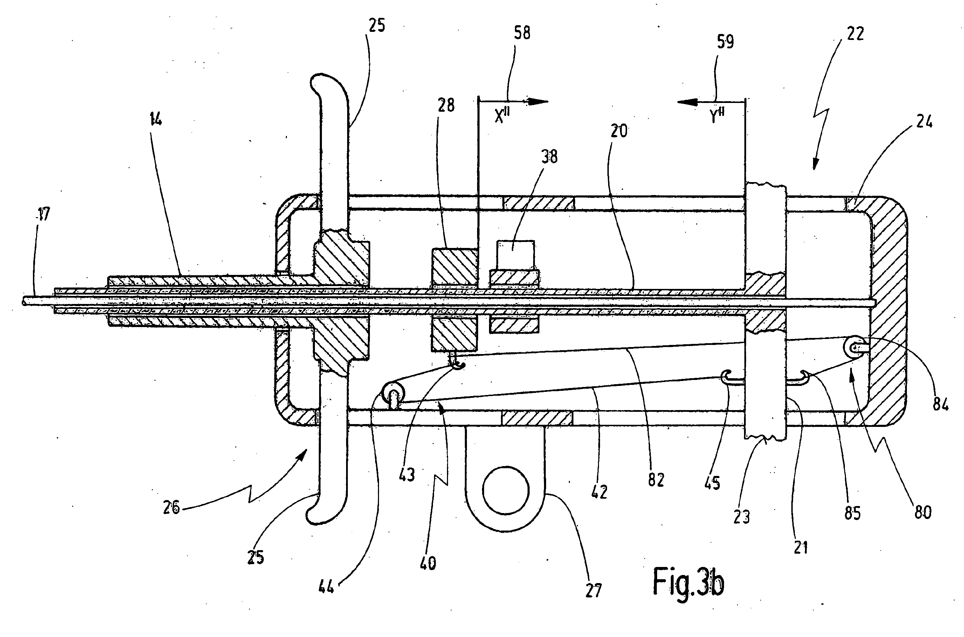

In Fig. 3a und 3b ist eine weitere Ausführungsform des erfindungsgemäßen Einführsystems dargestellt, wobei - wie in Fig. 2a und 2b - lediglich der Griff 22 der Vorrichtung in einem Längsschnitt dargestellt ist. Dabei wurden auch hier für die gleichen elemente die gleichen Bezugszeichen wie in Fig. 1 und Fig. 2a und 2b verwendet. Danach weist auch der Griff 22 in Fig. 3a und 3b einen Zuggriff 26 auf, der in der in Fig. 3a und 3b abgebildeten Position am distalen Ende im Gehäuse 24 des Griffs 22 verschiebbar angebracht ist. Im Gehäuse 24 ist ferner das Schiebeelement 20 untergebracht, der - wiederum wie in Fig. 2a und 2b - durch den Zuggriff hindurch verschiebbar angeordnet ist. Der Zuggriff 26 ist fest mit dem Hüllschlauch 14 verbunden. Ferner befindet sich im Gehäuse 24 ein bewegliches Element 28, das entlang des Schiebeelements 20 verschiebbar angebracht ist, oder durch das hindurch das Schiebeelement 20 verschiebbar angeordnet ist. Ferner weist die Vorrichtung in Fig. 3a und 3b wie bereits auch diejenige aus Fig. 2a und 2b ein Stoppelement 38 auf, welches in dem Gehäuse 24 entfernbar angebracht ist.

Der Griff 22 weist in seinem Gehäuse 24 ferner ein Umlenkgetriebe 40 auf, welches einen Zugfaden 42 umfasst, der mit einem seiner Enden am beweglichen Element 28 über ein Hakenelement 43 befestigt ist. Der Zugfaden 42 wird dann ausgehend vom beweglichen Element 28 über ein erstes Umlenkelement 44 geführt, welches sich im distalen Bereich des Gehäuses 24 des Griffs 22 befindet. Über dieses erste Umlenkelement 44 wird der Zugfaden 42 dann zum Schiebeelement 20 geführt, wo er mit seinem anderen Ende über ein weiteres Hakenelement 45 befestigt ist.

Auf diese Weise ist das bewegliche Element 28 mit dem Schiebeelement 20 gekoppelt. Die Pfeile 58 und 59 geben die Bewegungsrichtung und Weglänge des beweglichen Elements 28 (X) und des Schiebeelements 20 (Y) an. So ist zu erkennen, dass bei einer Bewegung des beweglichen Elements 28 in eine proximale Richtung, wie durch den Pfeil 58 dargestellt, das Schiebeelement 20 aufgrund der Kopplung mit dem beweglichen Element 28 über ein Umlenkgetriebe 40 in distale Richtung verschoben wird, was durch den Pfeil 59 dargestellt ist.

Mit dem in Fig. 3a und 3b gezeigten Umlenkgetriebe, bzw. mit dem in Fig. 3a und 3b dargestellten Einführsystem lässt sich danach aufgrund der besonderen Anordnung von Zugfaden 42 und Umlenkgelenk 44 ein Übersetzungsverhältnis von X'/Y' = 1 erzielen. Dabei ist mit X' der Weg des beweglichen Elements 28 bezeichnet, mit Y' der Weg des proximalen Endes des Schiebeelements 20, somit des Flansches 21.

Auch die in Fig. 3a gezeigte Ausführungsform kann - wie diejenige aus Fig. 2a - ein weiteres Getriebe 80 aufweisen, das die gleiche Funktion wie das Getriebe 70 aus Fig. 2b hat, nämlich um dem Anwender die Möglichkeit zu bieten, den Stent durch Zusammenschieben des beweglichen Elements 28 mit dem Flansch 21 freizusetzen. Die Ausführungsform mit dem zweiten Getriebe ist in Fig. 3b gezeigt.

In Fig. 3b ist dargestellt, dass das Gehäuse 24 ein Getriebe 80 aufweist, welches einen Zugfaden 82 umfasst, der mit einem seiner Enden am beweglichen Element 28 über das Hakenelement 43 befestigt ist. Der Zugfaden 82 wird dann ausgehend vom beweglichen Element 28 über ein Umlenkelement 84 geführt, welches sich im proximalen Bereich des Gehäuses 24 des Griffs 22 befindet. Über dieses Umlenkelement 84 wird der Zugfaden 82 dann zum Schiebeelement 20 geführt, wo er mit seinem anderen Ende über ein weiteres Hakenelement 85 befestigt ist.

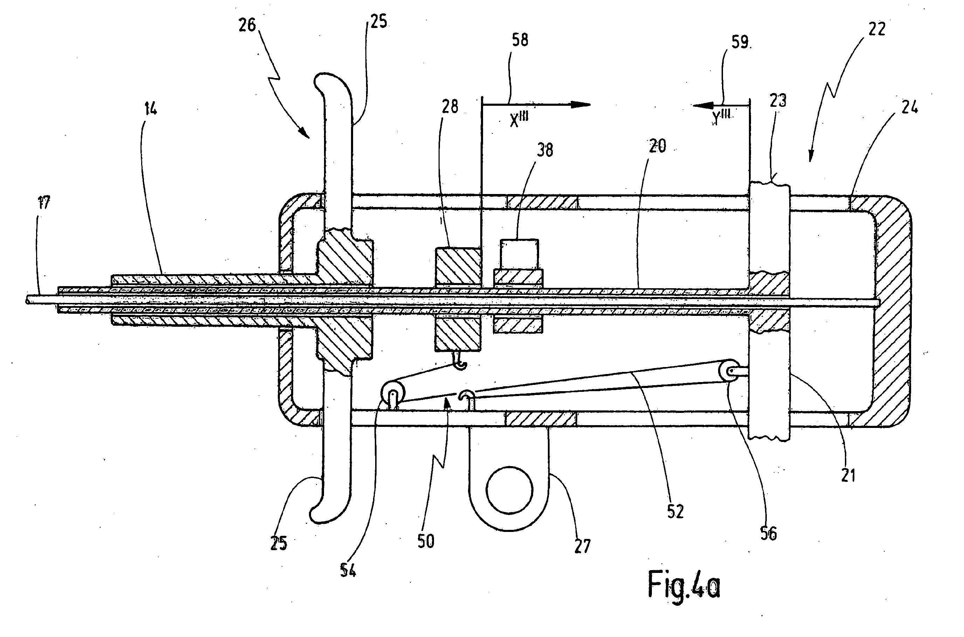

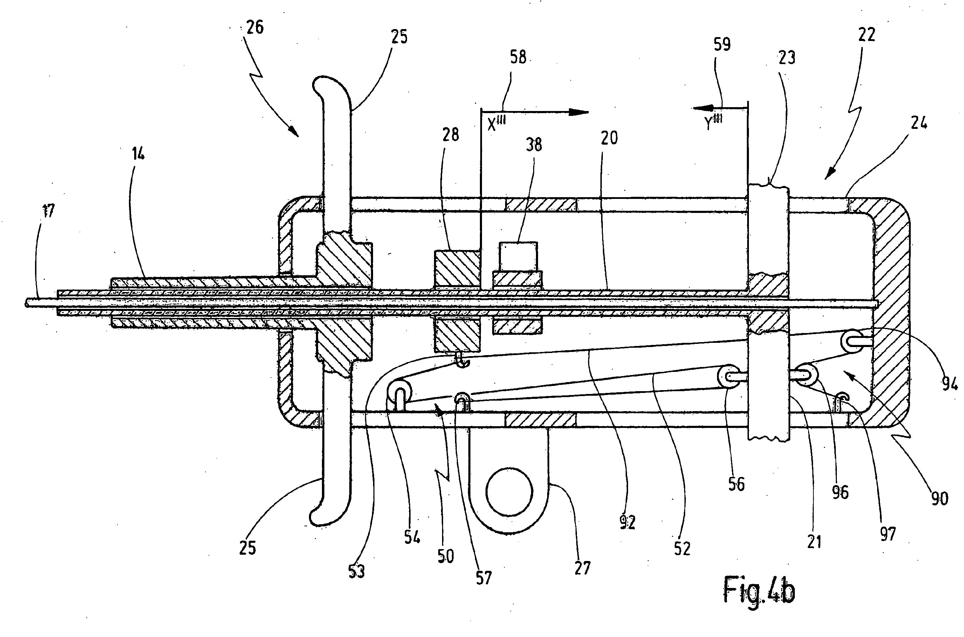

In Fig. 4a und 4b ist eine weitere Ausführungsform des erfindungsgemäßen Einführsystems gezeigt, bzw. eine besondere Ausgestaltung des Umlenkgetriebes, das im Gehäuse 24 der Vorrichtung vorgesehen ist.

In Fig. 4a und 4b wurden - wie in den Fig. 1 bis 3 - die gleichen Elemente mit den gleichen Bezugszeichen versehen. So ist auch in Fig. 4a und 4b der Längsschnitt durch das Gehäuse 24 des Griffes 22 dargestellt, wobei der Griff 22 einen im Gehäuse 24 verschiebbaren Zuggriff 26 aufweist, der mit dem Schlauch 14 fest verbunden bzw. gekoppelt ist. Im Gehäuse 24 ist ferner ein Schiebeelement 20 geführt, das an seinem proximalen Ende einen Flansch 21 aufweist, dessen Enden aus dem Gehäuse 24 des Griffs 22 herausragen und eine geriffelte Oberfläche 23 aufweisen. Mit dieser geriffelten Oberfläche 23 lässt sich - wie auch bereits in den Fig. 1 bis 3 - das Schiebeelement 20 gegebenenfalls per Hand greifen und sowohl in proximale also auch in distale Richtung verschieben. Entlang des Schiebeelements 20 ist ein bewegliches Element 28 verschiebbar angebracht, bzw. durch das bewegliche Element 28 und den Zuggriff 26 hindurchgeführt. Das bewegliche Element 28 ist mit dem Schiebeelement 20 über ein Umlenkgetriebe 50 gekoppelt.

Das Umlenkgetriebe 50 weist einen Zugfaden 52 auf, der mit einem seiner Enden über ein Hakenelement 53 am beweglichen Element 28 befestigt ist. Der Zugfaden 52 wird über ein erstes Umlenkelement 54 geführt, welches sich im distalen Bereich des Gehäuses 24 des Griffs 22 befindet. Von diesem ersten Umlenkgelenk 54 wird der Zugfaden 52 zu einem zweiten Umlenkgelenk 56 geführt, welches sich am proximalen Ende des Schiebeelements 20 befindet. In Fig. 4a und 4b ist gezeigt, dass sich das zweite Umlenkgelenk 56 dabei am Flansch 21 des Schiebeelements 20 befindet. Von diesem zweiten Umlenkgelenk 56 wird der Zugfaden 52 wieder in den distalen Bereich des Gehäuses 24 geführt, wo er mit seinem zweiten Ende an einem weiteren Hakenelement 57 befestigt ist.

Auch in Fig. 4a und 4b ist zu erkennen, dass eine Verschiebung des beweglichen Elements 28 in die proximale Richtung, wie durch den Pfeil 58 dargestellt, eine Verschiebung des Schiebeelements 20 in distaler Richtung bewirkt, was durch den Pfeil 59 gezeigt ist. Die Wegstrecke X', die das bewegliche Element 28 dabei zurücklegt, und die Wegstrecke Y', die das proximale Ende des Schiebeelements 20 zurücklegt, stehen bei der Vorrichtung in Fig. 4 in einem Übersetzungsverhältnis von X'/Y' = 2. Dies bedeutet, dass das bewegliche Element 28 einen doppelt so langen Weg zurücklegt wie das Schiebeelement 20.

In Fig. 4b ist ferner die den Fig. 2b und 3b entsprechende Ausführungsform mit einem weiteren Getriebe gezeigt. Das weitere Getriebe 90 weist einen Zugfaden 92 auf, der mit einem seiner Enden über das Hakenelement 53 am beweglichen Element 28 befestigt ist. Der Zugfaden 92 wird über ein Umlenkelement 94 geführt, welches sich im proximalen Bereich des Gehäuses 24 des Griffs 22 befindet. Von diesem Umlenkgelenk 94 wird der Zugfaden 92 zu einem Umlenkgelenk 96 geführt, welches sich am proximalen Ende des Schiebeelements 20 befindet. Das Umlenkgelenk 96 ist am Flansch 21 des Schiebeelements 20 angebracht. Von diesem Umlenkgelenk 96 wird der Zugfaden 92 wieder in den proximalen Bereich des Gehäuses 24 geführt, wo er mit seinem zweiten Ende an einem weiteren Hakenelement 97 befestigt ist. Auch die Umlenkelemente 94 und 96 sind in Fig. 4b als Rollen ausgebildet.

Alle in den Fig. 1 bis 4 dargestellten Ausführungsformen der erfindungsgemäßen Vorrichtung weisen ein außerhalb am Gehäuse 24 des Griffs 22 angebrachtes Greifelement 27 auf, das in den Fig. 1 bis 4 als Daumenloch ausgebildet ist. Über das Greifelement 27 kann der Griff 22 und damit das gesamte Einführsystem sicher in der Hand des Anwenders fixiert werden.

Ferner ist bei allen in den Fig. 1 bis 4 dargestellten Ausführungsformen im Gehäuse 24 ein Stoppelement 38 vorgesehen, welches das direkte Anstoßen des beweglichen Elements 28 mit dem proximalen Ende des Schiebeelements 20, im vorliegenden Falle also mit dem Flansch 21, verhindern kann. Das Schiebeelement 20 ist dabei in allen Ausführungsformen durch das Stoppelement 38, das bewegliche Element 28 und den Zuggriff 26 hindurchgeführt. Alle Elemente, also das Schiebeelement 20, das bewegliche Element 28, das Stoppelement 38 und der Zuggriff 26, können dabei gegeneinander verschoben werden, bzw. sind verschiebbar gegeneinander angebracht, wobei - wie beschrieben - das Schiebeelement 20 durch die anderen Elemente verschiebbar hindurchgeführt ist.

Das Stoppelement 38 ist dabei lösbar im Gehäuse 24 angebracht und kann zur letztendlichen Freisetzung des Stents 12 und zum Herbeiführen des direkten Kontakts zwischen beweglichem Element 28 und proximalem Ende des Schiebeelements 20, also dem Flansch 21, herausgezogen werden.

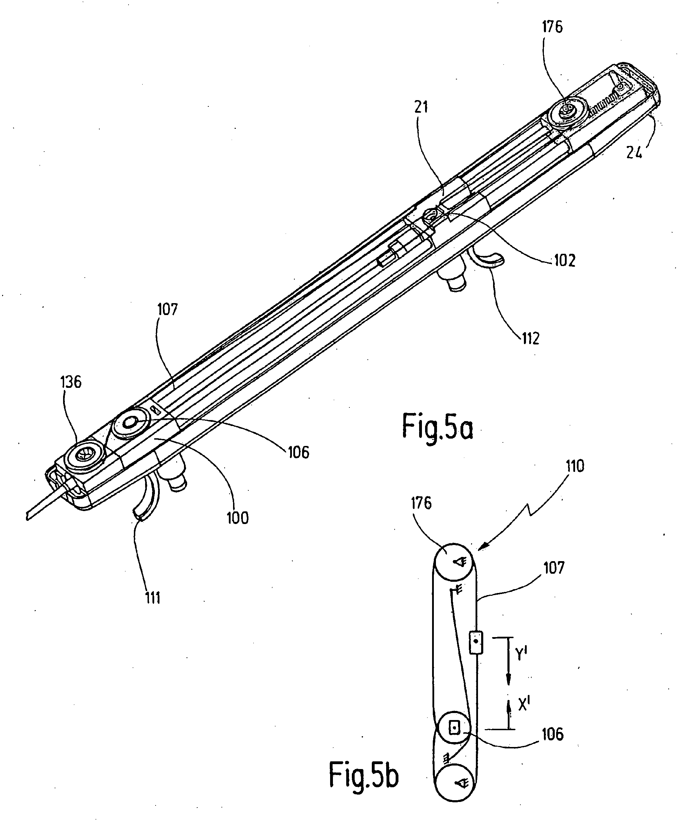

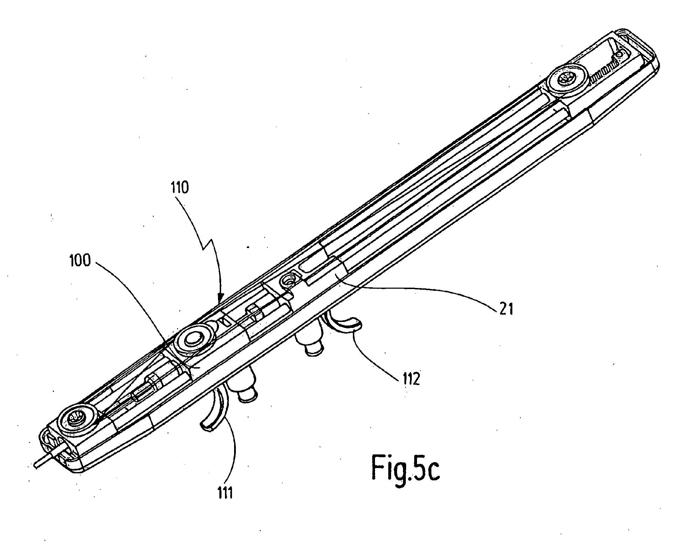

Die Fig. 5 bis 7 zeigen Weiterbildungen einer anderen Ausführungsform der erfindungegemäßen Vorrichtung. Bei diesen Weiterbildungen wurden die in der zuvor beschriebenen Ausführungsform vorgesehenen Umlenkelemente/Rollen und Hakenelemente sowie Zugfäden vereinigt. Ferner ist der in den Fig. 2 bis 4 beschriebene Zuggriff mit dem beweglichen Element als ein Bauteil vereinigt, beide Elemente bilden nun in den Fig. 5 bis 7 ein Zugelement bzw. einen Zugflansch.

In den Fig. 5 bis 7 wurde zur Bezeichnung von Elementen, die gleich zu den in den Fig. 2 bis 4 dargestellten Elementen sind, die gleichen Bezugszeichen verwendet. Außerdem werden für Elemente die in den Fig. 5 bis 7 gleichartig sind, die gleichen Bezugszeichen verwendet.

In Fig. 5a ist mit 22 insgesamt der Griff der Weiterbildung der erfindungegemäßen Vorrichtung bezeichnet, der ein Gehäuse 24 aufweist. Zur Darstellung der in dem Gehäuse 24 vorgesehenen Elemente ist das Gehäuse 24 in Fig. 5 teilweise, hier hälftig, entfernt. Mit 100 ist das Zugelement bezeichnet, das hier in Form eines Flansches ausgebildet ist. Mit 110 ist insgesamt das Umlenkgetriebe dieser Ausführungsform bezeichnet. Das Zugelement 100 ist direkt mit dem Hüllschlauch für den freizusetzenden Stent verbunden (letztere Elemente nicht gezeigt). Das Zugelement 100 weist über das Gehäuse 24 hinausragende Elemente auf, wobei mit 111 ein Element in Form eines Abzugs bezeichnet ist. Das Zugelement 100 ist in der dargestellten Ausführungsform als Flansch ausgebildet und weist eine drehbare Rolle 106 auf, die flach auf dem Zugelement 100 angebracht ist und als Umlenkelement dient.

Wie in den Fig. 2 bis 4 ist in Fig. 5a mit 21 das als Flansch ausgebildete proximale Ende des Schiebeelements 20 dargestellt. Der Flansch 21 weist hier ein Klemmelement 102 auf, das einen Zugfaden 107 an dem Flansch 21 fixiert. Der Flansch weist wie das Zugelement 100 Elemente auf, die über das Gehäuse 24 hinausragen, wobei auch hier ein Element 112 als Abzug ausgebildet ist. Am proximalen und am distalen Ende des Gehäuses 24 sind jeweils weitere Umlenkelemente 136, 176 vorgesehen, die nach Art einer feststehenden Rolle ausgebildet und flach liegend angebracht sind. Die Umlenkelemente 136 und 176 dienen auch als Befestigung der Enden des Zugfadens 107. Der Zugfaden 107 wird, ausgehend vom distalen Ende des Gehäuses 24, an dem sein eines Ende befestigt ist, über die Rolle 106 geführt, die am Zugelement 100 vorgesehen ist. Anschließend ist der Zugfaden 107 um das Umlenkelement 136 gelegt, das sich am distalen Ende des Gehäuses 24 befindet. Der Zugfaden 107 verläuft weiter an das proximale Ende des Gehäuses 24 und ist dort um das Umlenkelement 176 geführt. Der Zugfaden 107 verläuft weiter zum Flansch 21, bzw. durch das am Flansch 21 vorgesehene Klemmelement 102 und wieder distalen Ende des Gehäuses 24, wo er mit seinem zweiten Ende befestigt ist. Über das Klemmelement 102 wird der Zugfaden 107 am Flansch 21 fixiert. Der Verlauf des Zugfadens 107 ist in Fig. 5b schematisch dargestellt.

Die Befestigung der Enden des Zugfadens 107 kann bspw. durch am Umlenkelement vorgesehene Mittel erfolgen.

In der Startposition, also vor Freisetzung des Stents, befindet sich das Zugelement 100 im Bereich des distalen Endes des Gehäuses 24, mit einer gewissen Distanz entfernt vom proximalen Ende des Schiebeelements 21. Bei einer Betätigung des Abzugs 111 oder 112 oder - zur einfacheren Handhabung - beider Abzüge 111, 112 wird das Zugelement 100 in proximale Richtung, d.h. hin zum Anwender geführt. Dies ist in Fig. 5c gezeigt, aus der offenbar ist, dass sich bei Betätigung des Abzuges 111 Zugelement 100 und das proximales Ende des Schiebeelements 20, also der Flansch 21, aufeinander zu bewegen. Aufgrund der Kopplung des Zugelements 100 mit dem Schiebeelement 20 über die Elemente des Umlenkgetriebe .

Aus den Fig. 5b und 5c ist ersichtlich, dass mit der hierin gezeigten Ausführungsform ein Übersetzungsverhältnis X'/Y' von 0,5 erreicht wird, wobei X' der Weg des beweglichen Elements 28 bzw. Zugelements 100 bezogen auf den Weg Y' des Flansches 21 des Schiebeelements 20 ist.

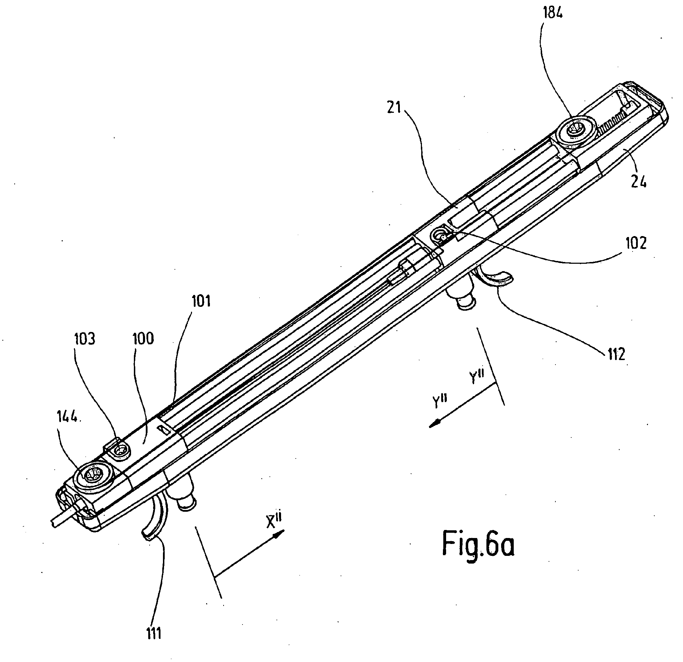

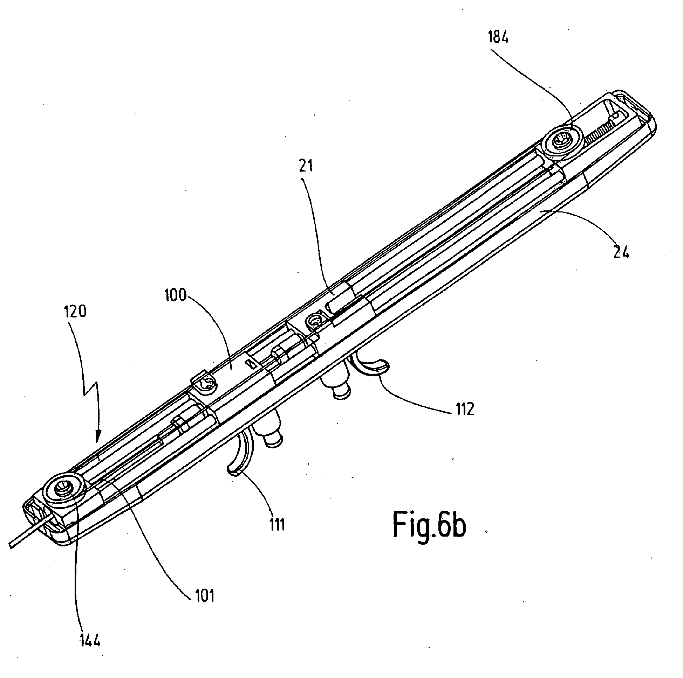

In Fig. 6 ist eine andere Weiterbildung einer Ausführungsform der erfindungsgemäßen Vorrichtung gezeigt, die der in Fig. 5 gezeigten Ausführungsform ähnlich ist, wobei hier das Umlenkgetriebe insgesamt mit 120 bezeichnet ist. In Fig. 6 ist ebenfalls zur Darstellung der in dem Gehäuse 24 vorgesehenen Elemente ist das Gehäuse 24 in Fig. 5 teilweise, hier hälftig, entfernt. Das flanschförmige Zugelement 100, das ebenfalls mit dem (Hüll-) Schlauch gekoppelt ist, weist in dieser Ausführungsform kein Umlenkelement auf, sondern vielmehr ein Klemmelement 103, in das ein Zugfaden 101 eingeklemmt und am Zugelement 100 dadurch fixiert wird. Der Zugfaden 101 ist in Fig. 6 umlaufend ausgebildet und verläuft um ein Umlenkelement 144 am distalen Ende des Gehäuses 24 und um ein Umlenkelement 184 am proximalen Ende des Gehäuses 24. Über ein Klemmelement 102 ist der Zugfaden 101 ferner am Flansch 21 des Schiebeelements 20 fixiert. Zugelement 100 und Flansch 21 weisen jeweils ein Abzugselement 111, 112 auf, über das die Elemente betätigt werden können. Der Flansch 21 kann je nach geladener Stentlänge - mit Bezug auf Fig. 6a - mehr rechts oder mehr links liegen.

In Fig. 6a ist der Ausgangszustand dargestellt, also der Zustand des Einführsystems, bei dem der Stent noch nicht freigesetzt ist. Die Freisetzung erfolgt durch ein Betätigen des Abzugs 111 oder 112 oder beider Abzüge 111, 112, wodurch das Zugelement 100 und der Flansch 21, also das proximale Ende des Schiebeelements 20 aufeinander zu bewegt werden. Dadurch wird der Stent freigesetzt. Dieser Zustand ist in Fig. 6b gezeigt. Mit der in Fig. 6 gezeigten Ausführungsform kann ein Übersetzungsverhältnis X"/Y" von 1 erreicht werden, wobei X" der Weg des beweglichen Elements 28 bzw. Zugelements 100 bezogen auf den Weg Y" des Flansches 21 des Schiebeelements 20 ist.

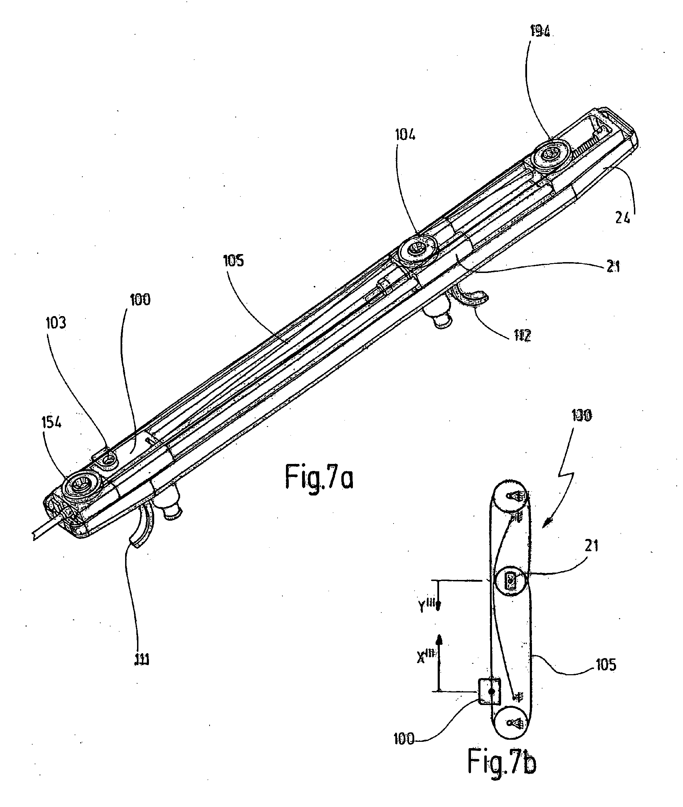

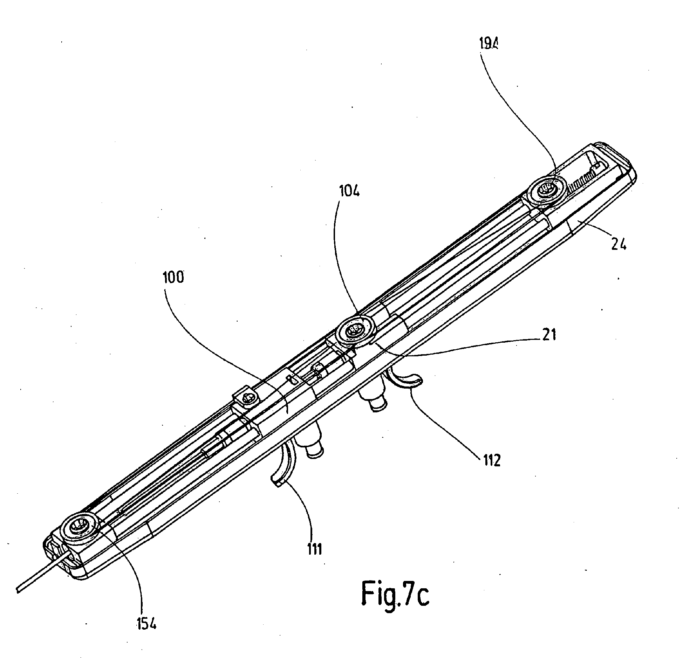

In Fig. 7 ist ebenfalls zur besseren Darstellung eine Hälfte des Gehäuses 24 entfernt. In Fig. 7 ist das Umlenkgetriebe mit 130 bezeichnet. Hier weist das Zugelement 100 ein Klemmelement 103 auf, über das ein Zugfaden 105 mit dem Zugelement 100 fixiert werden kann. Der Flansch 21 des Schiebeelements 20 weist eine drehbare Rolle 104 auf, die flach auf dem Flansch 21 befestigt ist. Das proximale und das distale Ende des Gehäuses 24 wiesen jeweils ein Umlenkelement in Form von feststehenden Rollen/Scheiben 154, 194 auf. Der Zugfaden 105 ist mit einem seiner Enden am distalen Ende des Gehäuses 24 befestigt und von dort um die auf dem Flansch 21 vorgesehene drehbare Rolle 104 geführt. Von dort wird er Zugfaden 105 wiederum um das am distalen Ende des Gehäuses 24 vorgesehene Umlenkelement 154 geführt, weiter zum am proximalen Ende des Gehäuses 24 vorgesehenen Umlenkelement 194, dann wiederum um die Rolle 104 des Flansches 21 und schließlich mit seinem anderen Ende am distalen Ende des Gehäuses 24 befestigt.

Die Befestigung der Enden des Zugfadens 105 kann bspw. durch am Umlenkelement vorgesehene Mittel erfolgen.

In Fig. 7b ist schematisch der Verlauf des Zugfadens gezeigt. In Fig. 7b ist - analog zu den Fig. 5c und 6b - wiederum der Zustand des Systems nach Freisetzung des Stents gezeigt, wonach durch eine Betätigung der Abzüge 111, 112 - oder einer der Abzüge 111, 112 - die beiden Elemente, Zugelement 100 und Flansch 21, aufeinander zu bewegt werden. Bei der in Fig. 7 gezeigten Ausführungsform kann ein Übersetzungsverhältnis X'''/Y''' von 2 erreicht werden, wobei X''' der Weg des beweglichen Elements 28 bzw. Zugelements 100 bezogen auf den Weg Y''' des Flansches 21 des Schiebeelements 20 ist.

Aus den Fig. 5 bis 7 geht hervor, dass das in distale Richtung bewegte Schiebeelement (Pusher) den Stent aus der Hülle schiebt, wobei gleichzeitig die Hülle durch die Kupplung mit dem Zugelement 100 durch dessen Bewegung in proximale Richtung auch aktiv vom Stent entfernt wird.

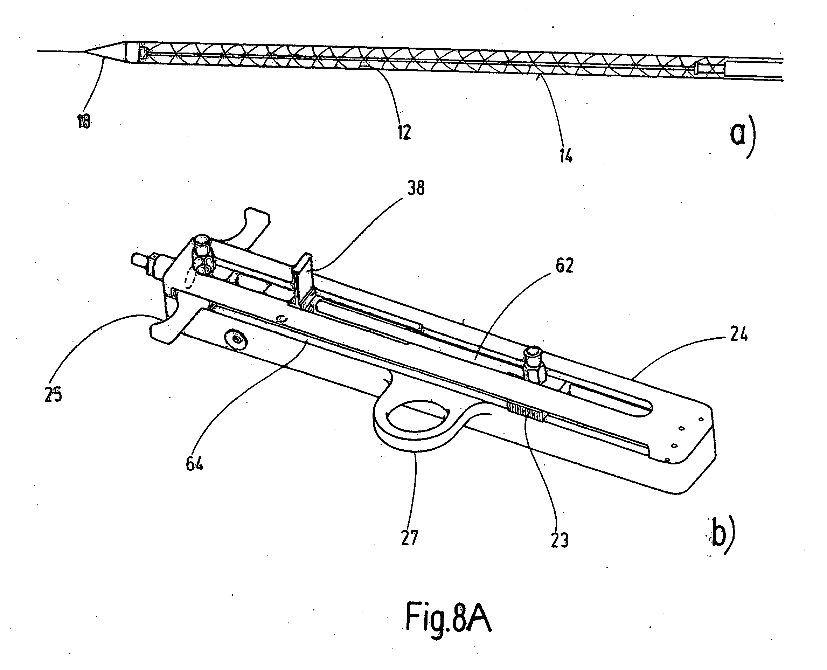

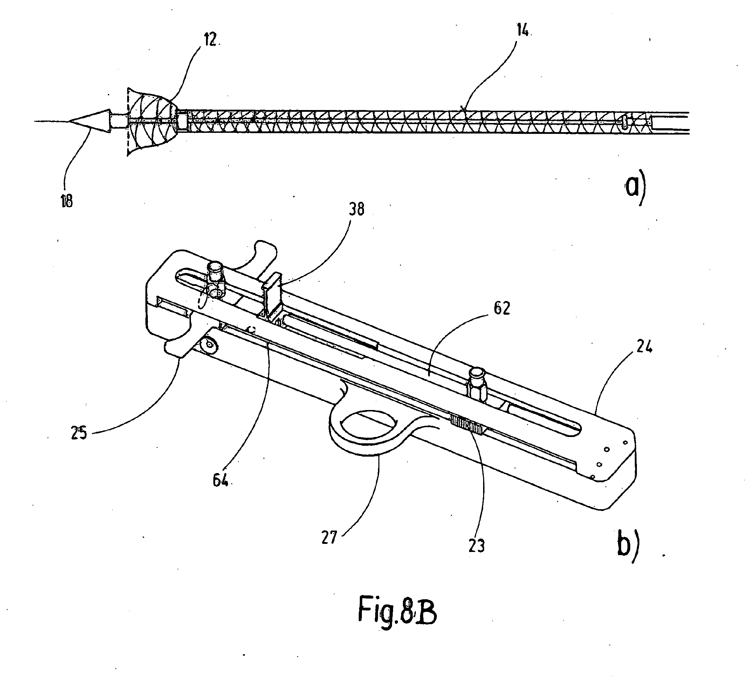

In den Fig. 8A bis 8E ist der Ablauf der Freisetzung eines Stents 12 durch ein in den Fig. 1 bis 7 dargestelltes Einführsystem schematisch dargestellt. Im Folgenden soll anhand der Fig. 8A bis 8E dieser Freisetzungsmechanismus näher erläutert werden, wobei der Freisetzungsmechanismus des Stents 12 bei allen in den Fig. 1 bis 7 dargestellten Ausführungsformen gleich ist. In den Fig. 8A bis 8E ist in der oberen Figurenhälfte, die jeweils mit a) bezeichnet ist, dabei jeweils der Freisetzungsstatus des Stents 12 gezeigt und in der unteren Figurenhälfte der Fig. 8A bis 8E, die mit b) bezeichnet sind, der entsprechende Status/Vorgang am Griff 22 des Einführsystems.

In den Fig. 8A bis 8E ist eine beispielhafte Ausgestaltung des Gehäuses 24 des Griffs 22 aller in den Fig. 1 bis 7 dargestellter Ausführungsformen der Vorrichtung dargestellt: Das Gehäuse 24 weist eine nutförmige, schienenartige Aussparung 62 auf, über welche die außerhalb des Gehäuses 24 liegenden Elemente des Zuggriffs 26, des beweglichen Elements 28 sowie des Schiebeelements 20 im Gehäuse 24 verschiebbar geführt sind. Solche Elemente sind bspw. Schrauben und Muttern, die für die Montierung des Zuggriffs 26, des beweglichen Elements 28 sowie des Schiebeelements 20 im bzw. am Gehäuse benötigt werden. Ferner wird in dieser Aussparung das Stoppelement 38 geführt, das teilweise aus dem Gehäuse 24 herausragt, wie in den Fig. 5 bis 8 dargestellt ist.

Ferner befindet sich seitlich des Gehäuses 24 des Griffs 22 eine längliche Aussparung 64, in welcher die Enden des Flanschs 21 des Schiebeelements 20 sowie die Greifflansche 25 des Zuggriffs 26 verschiebbar geführt sind.

Wie bereits weiter oben beschrieben, befindet sich zum Zwecke der Einführung eines Stents 12 in ein Körpergefäß dieser radial zusammengedrückt in einer Hülle bzw. einem Schlauch 14, um einen möglichst geringen Durchmesser zu bewirken. Dieser Zustand des Stents ist in den Fig. 8A bis 8E jeweils in der oberen Figurenhälfte dargestellt. Der Stent 12 drückt dabei aufgrund seiner Ausbildung als Metallstent mit einer bestimmten Kraft gegen den Schlauch 14. Der Schlauch 14 ist fest mit dem Zuggriff 26 gekoppelt, der in dem Gehäuse 24 des Griffs 22 des Einführsystems verschiebbar angebracht ist. Der Zuggriff 26 weist dabei zwei Griffflansche 25 auf, die aus dem Gehäuse 24 herausragen. Über die Griffflansche 25 kann der Zuggriff 26 einfach gegriffen werden.

Der Anwender, bspw. der behandelnde Chirurg, nimmt nun den Griff 22 in seine Hand und führt beispielsweise seinen Daumen in das Greifelement 27. Es versteht sich, dass das Greifelement 27 dabei jede Gestaltung aufweisen kann, die ein sicheres Festhalten eines oder mehrerer Finger am Griff 22 ermöglichen. In einem nächsten Schritt können dann beispielsweise Mittel- und Zeigefinger des Anwenders den Zuggriff 26, bzw. dessen Griffflansche 25, die aus dem Gehäuse 24 hervorragen, greifen. Es versteht sich, dass es nicht notwendig ist, dass stets Mittel- und Zeigefinger den Zuggriff umfassen; so kann beispielsweise auch die zweite Hand dazugenommen werden.

Mittel- und Zeigefinger werden zur Freisetzung des Stents 12 gegen den Daumen, also in proximale Richtung, gezogen. Dadurch wird der Zuggriff 26 zunächst um einen initialen Rückzugsweg Z des Hüllschlauches 14 zurückgezogen. Dies ist in Fig. 6 dargestellt. In Fig. 6a ist dargestellt, dass durch diese Bewegung das distale Ende des Stents 12 freikommt und sich aufgrund seiner Federwirkung distal der Stenose bzw. der Gefäßläsion an die Gefäßwand anlegt.

Durch ein weiteres Zurückziehen des Zuggriffs 26 wird nun neben dem Schlauch 14 auch das bewegliche Element 28 im Gehäuse 24 des Griffs 22 nach proximal mitgenommen. Dabei kann auch das Stoppelement 38 mitgenommen werden, welches entlang des Schiebeelements 20 beweglich im Gehäuse 24 angebracht ist.

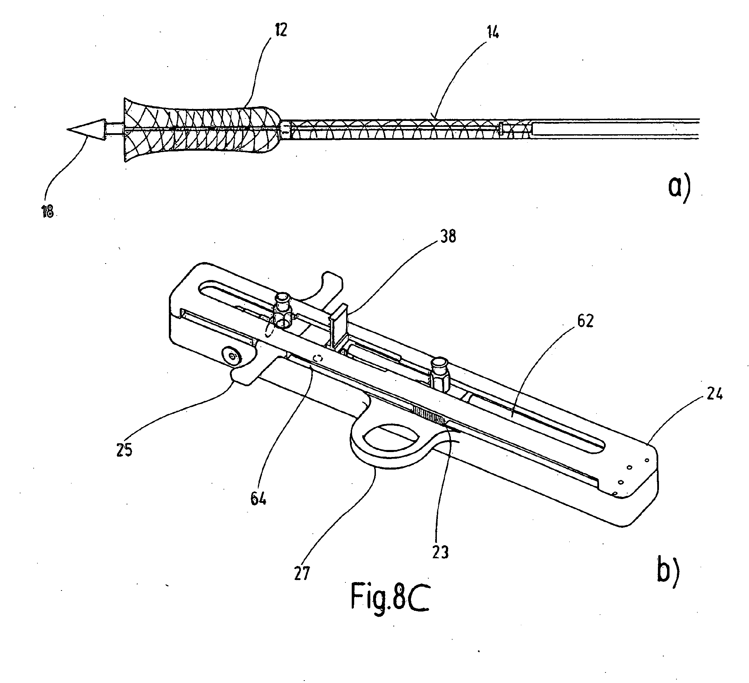

Das bewegliche Element 28 ist über ein Umlenkgetriebe 30, 40 oder 50 mit dem proximalen Ende des Schiebeelements 20, bzw. über dessen Flansch 21 mit diesem gekoppelt. Das Gehäuse 24 des Griffs 22 fungiert dabei als Festlagerbasis für das Umlenkgetriebe in den verschiedenen Ausführungsformen, wie sie hierin beschrieben sind. Die Kopplung zwischen dem beweglichen Element 28 mit dem Schiebeelement 20/dem Flansch 21 durch das Umlenkgetriebe 30, 40 oder 50 bewirkt dadurch eine Vorschubbewegung des Schiebeelements 20 nach distal. Dies ist in Fig. 8C dargestellt.

In Fig. 8C, obere Figurhälfte, mit a) bezeichnet, ist dargestellt, dass der Stent 12 aufgrund des Rückzugs des Schlauchs 14 und der Entgegenwirkung des Schiebeelements 20 nun schon zu einem größeren Teil freigesetzt ist.

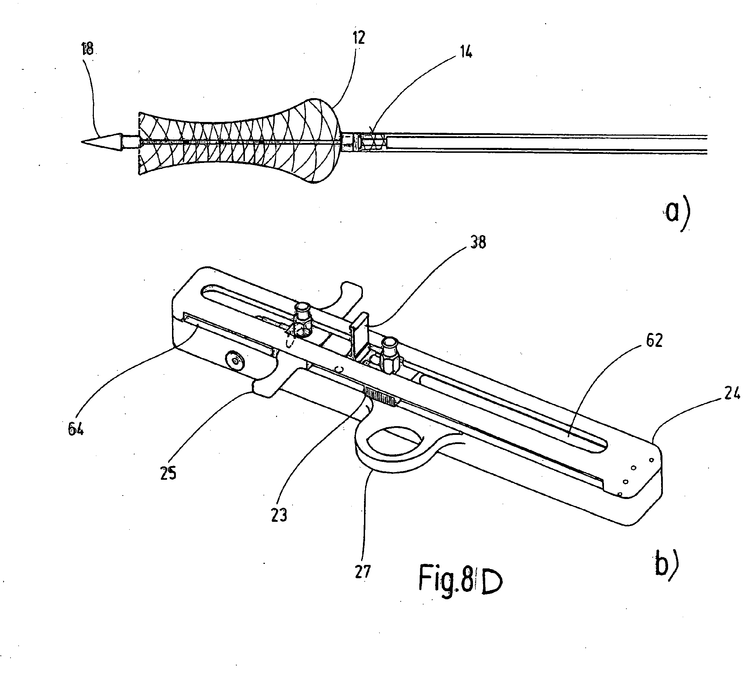

In den Fig. 8A bis 8E ist in weiteren Schritten dargestellt, dass der Zuggriff 26 und das mitgeführte bewegliche Element 28 bei einem weiteren Rückzug in eine proximale Richtung auf den Flansch 21 des Schiebeelements 20 treffen, bzw. von einem direkten Kontakt durch ein Stoppelement 38 abgehalten werden. Dadurch wird bewirkt, dass das bewegliche Element 28 und das proximale Ende des Schiebeelements 20, also der Flansch 21 des Schiebeelements 20, nicht direkt anschlagen. Dieser Zustand ist in Fig. 8D gezeigt.

Aufgrund dieser Ausgestaltung ist sichergestellt, dass ein ausreichender Teil des proximalen Ende des Stents 12 im Schlauch 14 liegen und am Schiebeelement 20 fixiert bleibt. Dies ist in Fig. 8a dargestellt, in der gezeigt ist, dass der Stent 12 an seinem proximalen Ende noch nicht vollkommen freigesetzt ist. In Fig. 8D, untere Figurenhälfte, ist parallel hierzu der "Zustand" des Griffs 22 gezeigt. Hier stehen Zuggriff 26, bewegliches Element 28, Stoppelement 38 und Flansch 21 des Schiebeelements 20 in dieser Reihenfolge in Kontakt. In dieser Phase kann der Stent 12, falls erforderlich, wieder vollständig in das Einführsystem zurückgezogen werden. Hierzu muss lediglich der Flansch 21 des Schiebeelements 20 an seinen außerhalb des Gehäuses 24 herausragenden Enden gegriffen und nach distal gezogen werden. Das Umlenkgetriebe 30, 40 oder 50 wird nun umgekehrt und drückt das bewegliche Element 28 samt Zuggriff 26 mit dem Hüllschlauch 14 nach distal. Der Stent 12 wird so schonend von der Gefäßwand abgehoben und gleichzeitig wieder in das Einführsystem und in den Schlauch 14 zurückgezogen.

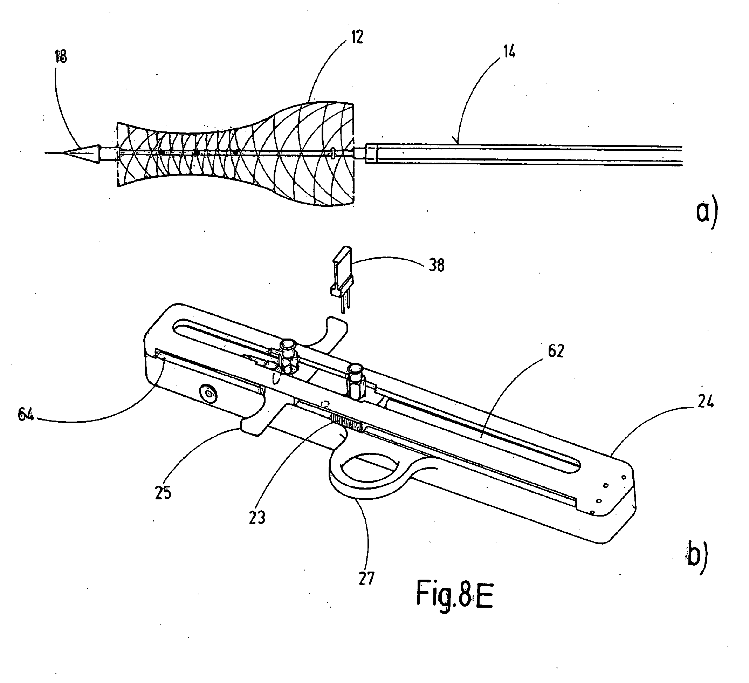

Soll der Stent 12 ganz freigesetzt werden, so wird das Stoppelement 38 zwischen dem beweglichen Element 28 und dem Flansch 21 des Schiebeelements 20 herausgezogen. Über den Zuggriff 26 kann dann das bewegliche Element 28 vollständig nach proximal bewegt werden. Dieser Schritt ist in Fig. 8E gezeigt. Aus Fig. 8E, obere Figurenhälfte, ist ersichtlich, dass der Stent 12 nun vollkommen freigesetzt ist, wobei parallel in Fig. 8E der Griff 22 gezeigt ist, bei dem das Stoppelement 38 entfernt wurde.

Der Führungsdraht 16 kann nun - bei Entfernung des Einführungssystems aus dem Gefäß - durch den aufgeweiteten Stent hindurch vorsichtig nach proximal herausgezogen werden.

Neben der Wahl von einer der Getriebevarianten, die hinsichtlich der Übersetzungskräfte und -verhältnisse sinnvoll einsetzbar sind, kann wie erwähnt auch der initiale Rückzugsweg Z des Hüllschlauches 14 variiert und dem jeweiligen Stentdurchmesser und Flechtwinkel angepasst werden. Dadurch können auch irrationale Zahlen als Gesamtübersetzungsverhältnisse (Z+X) /Y ≥ 0,5 ("größer gleich") zwischen Rückzugsweg des Hüllschlauches 14 und Vorwärtsbewegung des Schiebeelements 20 ermöglicht werden.

## Patentansprüche

1. Vorrichtung (10) zum Einführen eines selbstexpandierenden Stents (12) in ein Körpergefäß, mit:

   - einem Schlauch (14), der in einem distalen Abschnitt den Stent (12) radial zusammengedrückt hält,
   - einem in dem Schlauch (14) geführten Schiebeelement (20) mit einem proximalen und einem distalen Ende,
   - einem Griff (22), der ein Gehäuse (24) aufweist, über welches das Schiebeelement (20) am Griff (22) verschiebbar befestigt ist,
   - einem in dem Schiebeelement (20) geführten Stentträger mit Spitze (18), welcher über den Griff (22) fest an der Vorrichtung (10) angebracht ist,

   wobei ein in dem Gehäuse (24) des Griffs (22) geführtes bewegliches Element (28; 100) vorgesehen ist, das derart mit dem proximalen Ende des Schiebeelements (20) gekoppelt ist, dass durch eine Bewegung des beweglichen Elements (28; 100) in die proximale Richtung gleichzeitig das Schiebeelement (20) in die distale Richtung, und der Schlauch (14) in die proximale Richtung gezogen wird, **dadurch gekennzeichnet, dass** das bewegliche Element (28; 100) mit dem Schiebeelement (20) über ein im Griff (22) vorgesehenes Seilrollenumlenkgetriebe (30; 40; 50; 110; 120; 130) gekoppelt ist.

2. Vorrichtung nach Anspruch 1, **dadurch gekennzeichnet, dass** das bewegliche Element (28; 100) mit dem im Gehäuse liegenden proximalen Ende des Schiebeelements (20) über ein im Griff (22) vorgesehenes Seilrollenumlenkgetriebe (30; 40; 50; 110; 120; 130) gekoppelt ist.

3. Vorrichtung nach einem der Ansprüche 1 oder 2, **dadurch gekennzeichnet, dass** im Gehäuse (24) ferner ein Zuggriff (26) vorgesehen ist, der mit dem Schlauch (14) gekoppelt ist.

4. Vorrichtung nach Anspruch 3, **dadurch gekennzeichnet, dass** das Seilrollenumlenkgetriebe einen Zugfaden (32) aufweist, der mit dem einen seiner Enden am distalen Teil des Gehäuses (24) befestigt ist, der ferner über ein erstes, am beweglichen Element (28) vorgesehenes Umlenkelement (34) und über ein zweites, im distalen Ende des Gehäuses

(24) vorgesehenes Umlenkelement (36) zum Schiebeelement (20) geführt und mit seinem zweiten Ende am Schiebeelement (20) befestigt ist.

**5.** Vorrichtung nach Anspruch 3, **dadurch gekennzeichnet, dass** das Seilrollenumlenkgetriebe einen Zugfaden (42) aufweist, der mit dem einen seiner Enden am beweglichen Element (28) befestigt ist, der ferner über ein am distalen Ende des Gehäuses (24) vorgesehenes Umlenkelement (44) zum Schiebeelement (20) geführt und mit seinem zweiten Ende am Schiebeelement (20) befestigt ist.

**6.** Vorrichtung nach Anspruch 3, **dadurch gekennzeichnet, dass** das Seilrollenumlenkgetriebe einen Zugfaden (52) aufweist, der mit dem einen seiner Enden am beweglichen Element (28) befestigt ist, der ferner über ein erstes, am distalen Ende des Gehäuses (24) vorgesehenes Umlenkelement (54) zum Schiebeelement (20) geführt ist, und der über ein zweites, am Schiebeelement (20) vorgesehenes Umlenkelement wieder (56) zum distalen Ende des Gehäuses (24) geführt und mit seinem zweiten Ende am distalen Ende des Gehäuses (24) befestigt ist.

**7.** Vorrichtung nach einem der Ansprüche 1 bis 6, **dadurch gekennzeichnet, dass** ein zweites Getriebe (70; 80; 90) vorgesehen ist, das als Gegengetriebe des Seilrollenumlenkgetriebes arbeitet.

**8.** Vorrichtung nach einem der Ansprüche 1 bis 7, **dadurch gekennzeichnet, dass** der Griff (22) ferner ein Greifelement (39) aufweist, das fest am Gehäuse (24) angebracht ist.

**9.** Vorrichtung nach einem der Ansprüche 1 oder 2, **dadurch gekennzeichnet, dass** das bewegliche Element (28) als flanschförmiges Zugelement (100) ausgebildet ist, das auch mit dem Schlauch (14) gekoppelt ist.

**10.** Vorrichtung nach Anspruch 9, **dadurch gekennzeichnet, dass** das Seilrollenumlenkgetriebe einen Zugfaden (107) aufweist, der mit dem einen seiner Enden am distalen Teil des Gehäuses (24) befestigt ist, der ferner über ein erstes, am Zugelement (100) vorgesehenes Umlenkelement (106) und über ein zweites, im distalen Ende des Gehäuses (24) vorgesehenes Umlenkelement (136), zu einem am proximalen Ende des Gehäuses (24) vorgesehenen Umlenkelement (176) geführt ist, und anschließend über ein am proximalen Ende des Schiebelements (20) vorgesehenes Mittel (102) zum Fixieren des Zugfadens (107) am proximalen Ende des Schiebeelements (20) wieder um das Umlenkelement (106) geführt ist und mit seinem zweiten Ende am proximalen Ende des Gehäuses (24) befestigt ist.

**11.** Vorrichtung nach Anspruch 9, **dadurch gekennzeichnet, dass** das Seilrollenumlenkgetriebe einen umlaufenden Zugfaden (101) aufweist, der über ein am distalen Ende des Gehäuses (24) vorgesehenes Umlenkelement (144), über ein am Zugelement (100) vorgesehenes erstes Mittel (103) zum Fixieren des Zugfadens (107) am Zugelement (100), über ein am proximalen Ende des Gehäuses (24) vorgesehenes Umlenkelement (184) und über ein am proximalen Ende des Schiebeelements (20) vorgesehenes zweites Mittel (102) zum Fixieren des Zugfadens (101) am proximalen Ende des Schiebeelements (20) geführt ist.

**12.** Vorrichtung nach Anspruch 9, **dadurch gekennzeichnet, dass** das Seilrollenumlenkgetriebe einen Zugfaden (105) aufweist, der mit dem einen seiner Enden am distalen Ende des Gehäuses (24) befestigt ist und über ein am proximalen Ende des Schiebeelements (20) vorgesehenes Umlenkelement (104), über ein am distalen Ende des Gehäuses (24) vorgesehenes Umlenkelement (154), über ein am Zugelement (100) vorgesehenes erstes Mittel (103) zum Fixieren des Zugfadens (107) am Zugelement (100), und über ein am proximalen Ende des Gehäuses vorgesehenes Umlenkelement (194) geführt ist, und von dort wieder zum Umlenkelement (104), und der mit seinem zweiten Ende am proximalen Ende des Gehäuses (24) befestigt ist.

**13.** Vorrichtung nach einem der Ansprüche 1 bis 12, **dadurch gekennzeichnet, dass** sie ferner ein Stoppelement (38) aufweist, welches verschiebbar zwischen dem beweglichen Element (28) und dem Schiebeelement (20) zur Verhinderung des direkten Anstoßens des beweglichen Elements (28) mit dem proximalen Ende des Schiebeelements (20) vorgesehen ist

**Claims**

**1.** A device (10) for inserting a self-expanding stent (12) into a body vessel, with:

- a tube (14) which, in a distal portion, keeps the stent (12) radially compressed,
- a pushing element (20), which is guided in the tube (14) and has a proximal end and a distal end,
- a grip (22) having a housing (24) via which the pushing element (20) is secured displaceably on the grip (22),
- a stent carrier which is guided in the pushing element (20) and has a tip (18) which is mounted fixedly on the device (10) via the grip (22),

wherein a movable element (28; 100), which is guided in the housing (24) of the grip (22) is provided, which moveable element (28; 100) is coupled to the proximal end of the pushing element (20) in such a way that, by a movement of the movable element (28; 100) in the proximal direction, the pushing element (20) is at the same time pulled in the distal direction, and the tube (14) in the proximal direction, **characterized in that** the moveable element (28; 100) is coupled to the pushing element (20) via a cable pulley deflecting gear (30; 40; 50; 110; 120; 130) provided in the grip (22).

2. The device as claimed in claim 1, **characterized in that** the movable element (28; 100) is coupled to the proximal end of the pushing element (20) lying in the housing via a cable pulley deflecting gear (30; 40; 50; 110; 120; 130) provided in the grip (22).

3. The device as claimed in one of claims 1 or 2, **characterized in that** further a pull grip (26) is provided in the housing (24) and is coupled to the tube (14).

4. The device as claimed in claim 3, **characterized in that** the cable pulley deflecting gear has a tensioning thread (32) which is secured with one of its ends on the distal part of the housing (24), is further guided to the pushing element (20) via a first deflection element (34) provided on the movable element (28) and via a second deflection element (36) provided in the distal end of the housing (24), and is secured with its second end on the pushing element (20).

5. The device as claimed in claim 3, **characterized in that** the cable pulley deflecting gear has a tensioning thread (42) which is secured with one of its ends on the movable element (28), is further guided to the pushing element (20) via a deflection element (44) provided on the distal end of the housing (24), and is secured with its second end on the pushing element (20).

6. The device as claimed in claim 3, **characterized in that** the cable pulley deflecting gear has a tensioning thread (52) which is secured with one of its ends on the movable element (28), is further guided to the pushing element (20) via a first deflection element (54) provided on the distal end of the housing (24), is guided to the distal end of the housing (24) via a second deflection element (56) provided on the pushing element (20), and is secured with its second end on the distal end of the housing (24).

7. The device as claimed in one of claims 1 to 6, **characterized in that** a second gear (70; 80; 90) is provided which works as an opposing gear of the cable pulley deflecting gear.

8. The device as claimed in one of claims 1 through 7, **characterized in that** the grip (22) also has a gripping element (27) mounted fixedly on the housing (24).

9. The device as claimed in any of claims 1 or 2, **characterized in that** the movable element (28) is designed as a flange-shaped tensioning element (100), which is also coupled to the tube (14).

10. The device as claimed in claim 9, **characterized in that** the cable pulley deflecting gear has a tensioning thread (107) which is secured with one of its ends on the distal part of the housing (24), is further guided via a first deflection element (106) provided on the tensioning element (100) and via a second deflection element (136) provided in the distal end of the housing (24) to a deflection element (176) provided on the proximal end of the housing (24), and is then guided, via a means (102) provided on the proximal end of the pushing element (20) for fixing the tensioning thread (107) at the proximal end of the pushing element (20), back round the deflection element (106) and is secured with its second end on the proximal end of the housing (24).

11. The device as claimed in claim 9, **characterized in that** the cable pulley deflecting gear has a tensioning thread (101) which is guided via a deflection element (144) provided on the distal end of the housing (24), via a first means (103) provided on the tensioning element (100) for fixing the tensioning thread (107) on the tensioning element (100), via a deflection element (184) provided on the proximal end of the housing (24), and via a second means (102) provided on the proximal end of the pushing element (20) for fixing the tensioning thread (101) on the proximal end of the pushing element (20).

12. The device as claimed in claim 9, **characterized in that** the cable pulley deflecting gear has a tensioning thread (105) which is secured with one of its ends on the distal end of the housing (24) and is guided via a deflection element (104) provided on the proximal end of the pushing element (20), via a deflection element (154) provided on the distal end of the housing (24), via a first means (103) provided on the tensioning element (100) for fixing the tensioning thread (107) on the tensioning element (100), and via a deflection element (194) provided on the proximal end of the housing (24), and from there back to the deflection element (104), and is secured with its second end on the proximal end of the housing (24).

13. The device as claimed in one of claims 1 through 12, **characterized in that** it further comprises a stop element (38) which is displaceable between the movable element (28) and the pushing element (20) in order to prevent direct abutment of the movable element (28) with the proximal end of the pushing element (20)

## Revendications

1. Dispositif (10) servant à insérer un stent (12) intravasculaire auto-expansible, comportant :

   - un cathéter (14), lequel maintient de manière comprimée le stent (12) dans une section distale,
   - un élément coulissant (20) guidé dans le cathéter (14) avec une extrémité proximale et distale,
   - une poignée (22) qui présente un boîtier (24) par l'intermédiaire duquel l'élément coulissant (20) est fixé de manière à pouvoir être déplacé au niveau de la poignée (22),
   - un support de stent guidé dans l'élément coulissant (20), avec une pointe (18), lequel support de stent est apposé de manière fixe au niveau du dispositif (10) par l'intermédiaire de la poignée (22),

   sachant qu'un élément mobile (28 ; 100) guidés dans le boîtier (24) de la poignée (22) est prévu, lequel est couplé à l'extrémité proximale de l'élément coulissant (20) de telle manière qu'un déplacement de l'élément mobile (28 ; 100) dans la direction proximale permet d'entraîner de manière simultanée l'élément coulissant (20) dans la direction distale et le cathéter (14) dans la direction proximale, **caractérisé en ce que** l'élément mobile (28 ; 100) est couplé à l'élément coulissant (20) par l'intermédiaire d'un mécanisme de transmission par poulies (30 ; 40 ; 50 ; 110 ; 120 ; 130) prévu dans la poignée (22).

2. Dispositif selon la revendication 1, **caractérisé en ce que** l'élément mobile (28 ; 100) est couplé à l'extrémité proximale de l'élément coulissant (20), se trouvant dans le boîtier par l'intermédiaire d'un mécanisme de transmission par poulies (30 ; 40 ; 50 ; 110 ; 120 ; 130) prévu dans la poignée (22).

3. Dispositif selon l'une quelconque des revendications 1 ou 2, **caractérisé en ce qu'**est prévue en outre dans le boîtier (24) une poignée de traction (26), laquelle est couplée au cathéter (14).

4. Dispositif selon la revendication 3, **caractérisé en ce que** le mécanisme de transmission par poulies présente un fil de traction (32), dont l'une des extrémités est fixée à la partie distale du boîtier (24) qui est guidée en outre vers l'élément coulissant (20) par l'intermédiaire d'un premier élément de renvoi (34) prévu au niveau de l'élément mobile (28) et par l'intermédiaire d'un second élément de renvoi (36) prévu au niveau de l'extrémité distale du boîtier (24), et dont la seconde extrémité est fixée au niveau de l'élément coulissant (20).

5. Dispositif selon la revendication 3, **caractérisé en ce que** le mécanisme de transmission par poulies présente un fil de traction (42), dont l'une des extrémités est fixée au niveau de l'élément mobile (28), qui en outre est guidé vers l'élément coulissant (20) par l'intermédiaire d'un élément de renvoi (44) prévu au niveau de l'extrémité distale du boîtier (24), et dont la seconde extrémité est fixée au niveau de l'élément coulissant (20).

6. Dispositif selon la revendication 3, **caractérisé en ce que** le mécanisme de transmission par poulies présente un fil de traction (52), dont l'une des extrémités est fixée au niveau de l'élément mobile (28), lequel est en outre guidé vers l'élément coulissant (20) par l'intermédiaire d'un premier élément de renvoi (54) prévu au niveau de l'extrémité distale du boîtier (24) et qui est guidé à nouveau vers l'extrémité distale du boîtier (24) par l'intermédiaire d'un second élément de renvoi (56) prévu au niveau d'un élément coulissant (20) et dont la seconde extrémité est fixée au niveau de l'extrémité distale du boîtier (24).

7. Dispositif selon l'une quelconque des revendications 1 à 6, **caractérisé en ce qu'**un second mécanisme de transmission (70 ; 80 ; 90) est prévu, lequel fonctionne comme contre-mécanisme de transmission du mécanisme de transmission par poulies.

8. Dispositif selon l'une quelconque des revendications 1 à 7, **caractérisé en ce que** la poignée (22) présente en outre un élément de préhension (39), qui est apposé de manière fixe au niveau du boîtier (24).

9. Dispositif selon l'une quelconque des revendications 1 ou 2, **caractérisé en ce que** l'élément mobile (28) est réalisé en tant qu'élément de traction (100) en forme de bride, lequel est également couplé au cathéter (14).

10. Dispositif selon la revendication 9, **caractérisé en ce que** le mécanisme de transmission par poulies présente un fil de traction (107), dont l'une des extrémités est fixée au niveau de la partie distale du boîtier (24), qui en outre est guidé vers un élément de renvoi (176) prévu au niveau de l'extrémité proximale du boîtier (24) par l'intermédiaire d'un premier élément de renvoi (106) prévu au niveau de l'élément de traction (100) et par l'intermédiaire d'un second élément de renvoi (136) prévu à l'extrémité distale du boîtier (24), et qui pour finir est guidé à nouveau autour d'un élément de renvoi (106) par l'intermédiaire d'un moyen (102) servant à fixer le fil de traction (107) au niveau de l'extrémité proximale de l'élément coulissant (20), prévu au niveau de l'extrémité proximale de l'élément coulissant (20), et dont la seconde extrémité est fixée au niveau de l'extrémité proximale du boîtier (24).

**11.** Dispositif selon la revendication 9, **caractérisé en ce que** le mécanisme de transmission par poulies présente un fil de traction (101) rotatif, lequel est guidé par l'intermédiaire d'un élément de renvoi (144) prévu au niveau de l'extrémité distale du boîtier (24), par l'intermédiaire d'un premier moyen (103) prévu au niveau de l'élément de traction (100), servant à fixer le fil de traction (107) au niveau de l'élément de traction (100), par l'intermédiaire d'un élément de renvoi (184) prévu au niveau de l'extrémité proximale du boîtier (24), et par l'intermédiaire d'un second moyen (102) prévu au niveau de l'extrémité proximale de l'élément coulissant (20), servant à fixer le fil de traction (101) au niveau de l'extrémité proximale de l'élément coulissant (20).

**12.** Dispositif selon la revendication 9, **caractérisé en ce que** le mécanisme de transmission par poulies présente un fil de traction (105), dont l'une des extrémités est fixée au niveau de l'extrémité distale du boîtier (24) et qui est guidé par l'intermédiaire d'un élément de renvoi (104) prévu au niveau de l'extrémité proximale de l'élément coulissant (20), par l'intermédiaire d'un élément de renvoi (154) prévu au niveau de l'extrémité distale du boîtier (24), par l'intermédiaire d'un premier moyen (103) prévu au niveau de l'élément de traction (100), servant à fixer le fil de traction (107) au niveau de l'élément de traction (100), et par l'intermédiaire d'un élément de renvoi (194) prévu au niveau de l'extrémité proximale du boîtier, et de là à nouveau vers l'élément de renvoi (104), et dont la seconde extrémité est fixée au niveau de l'extrémité proximale du boîtier (24).

**13.** Dispositif selon l'une quelconque des revendications 1 à 12, **caractérisé en ce qu'**il présente en outre un élément d'arrêt (38), lequel est prévu de manière à pouvoir être déplacé entre l'élément mobile (28) et l'élément coulissant (20) afin d'éviter que l'élément mobile (28) ne heurte directement l'extrémité proximale de l'élément coulissant (20).

Fig.1

Fig.2a

EP 1 976 466 B1

Fig.2b

EP 1 976 466 B1

Fig.3a

Fig.3b

Fig.4a

EP 1 976 466 B1

23

Fig.4b

EP 1 976 466 B1

176

21

24

102

112

107

136

106

100

111

**Fig.5a**

110

176

107

Y'

X'

106

**Fig.5b**

Fig.5c

Fig.6a

Fig.6b

Fig.7a

Fig.7b

Fig.7c

Fig.8A

EP 1 976 466 B1

Fig.8B

EP 1 976 466 B1

EP 1 976 466 B1

12
14
18
a)

38
62
24
25
64
27
23

Fig.8C

b)

18　12　14

a)

38

64

25

23

27

62

24

Fig.8 D

b)

EP 1 976 466 B1

Fig.8E

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 10335649
- US 20030191516 A1
- US 5707376 A
- EP 1440671 A2